# EUROPÄISCHE PATENTSCHRIFT

(11) **EP 2 710 109 B1**
(45) Veröffentlichungstag und Bekanntmachung des Hinweises auf die Patenterteilung: **25.10.2017**
(21) Anmeldenummer: 11723389.0
(22) Anmeldetag: 19.05.2011
(51) Int. Cl.: C12M 1/34, G01N 33/487, C12M 3/00

(54) **VERFAHREN UND VERWENDUNG EINER VORRICHTUNG ZUR AUTOMATISIERTEN POSITIONSBESTIMMUNG EINES MIKROSYSTEMS ZUR MANIPULATION EINES SPHÄRISCHEN MIKRO-OBJEKTS**
METHOD AND UTILIZATION OF A DEVICE FOR AUTOMATICALLY DETERMINING THE POSITION OF A MICROSYSTEM FOR MANIPULATING A SPHERICAL MICRO-OBJECT
PROCÉDÉ ET UTILIZATION D'UN DISPOSITIF POUR LA DÉTERMINATION AUTOMATIQUE DE LA POSITION D'UN MICROSYSTÈME POUR LA MANIPULATION D'UN MICRO-OBJET SPHÉRIQUE

(43) Veröffentlichungstag der Anmeldung: 26.03.2014
(73) Patentinhaber: NMI Naturwissenschaftliches und Medizinisches Institut an der Universität Tübingen, 72770 Reutlingen (DE)
(72) Erfinder: DANKER, Timm, 72074 Tübingen (DE); GUENTHER, Elke, 74762 Reutlingen (DE)
(74) Vertreter: Witte, Weller & Partner Patentanwälte mbB
(86) Internationale Anmeldenummer: PCT/EP2011/058122
(87) Internationale Veröffentlichungsnummer: WO 2012/155973

(56) Entgegenhaltungen:
- WO-A2-02/03058
- US-A1- 2002 098 575
- US-B1- 6 470 226
- US-B1- 7 384 733

## Beschreibung

Die vorliegende Erfindung betrifft ein Verfahren zur automatisierten Bestimmung der relativen Position zwischen einer ersten Öffnung an einer ersten Mikrosystemkomponente, die vorzugsweise mit einem in der ersten Öffnung mündenden ersten Kanal versehen ist, und zumindest einer zweiten Öffnung an einer zweiten Mikrosystemkomponente, die vorzugsweise mit einem in der zweiten Öffnung mündenden zweiten Kanal versehen ist, bei dem sich die beiden Mikrosystemkomponenten zumindest im Bereich der Öffnungen in einem flüssigen Medium befinden.

Ferner betrifft die vorliegende Erfindung die Verwendung einer Vorrichtung zum automatisierten Kontaktieren von zumindest einem mikroskopischen Testobjekt, vorzugsweise einer biologischen Zelle, wobei das Testobjekt an einer ersten Öffnung immobilisiert und dann über eine zweite Öffnung kontaktiert wird, wobei die Vorrichtung mit zumindest einer ersten Mikrosystemkomponente, an der die erste Öffnung vorgesehen ist, die vorzugsweise mit einem in der ersten Öffnung mündenden ersten Kanal versehenen ist, zumindest einer zweiten Mikrosystemkomponente, an der die zweite Öffnung vorgesehen ist, die vorzugsweise mit einem in der zweiten Öffnung mündenden zweiten Kanal versehenen ist, und mit einem Reaktionsgefäß zur Aufnahme eines flüssigen Mediums versehen ist, wobei die beiden Mikrosystemkomponenten zumindest im Bereich der Öffnungen in das Medium eingetaucht sind.

Aus der US 2002/098575 A1 sind eine Vorrichtung und ein mit der Vorrichtung durchzuführendes Verfahren bekannt, um eine erste Mikrosystemkomponente mit Hilfe einer Führungsmanschette (guide collar) in einem flüssigen Medium automatisch so zu positionieren, dass sie ein an einer zweiten Mikrosystemkomponente immobilisiertes mikroskopisches Testobjekt kontaktiert.

Unter "Kontaktieren" wird im Zusammenhang mit der vorliegenden Erfindung sowohl der unmittelbare physische Kontakt mit der Oberfläche oder dem Inneren des mikroskopischen Testobjektes verstanden als auch das Applizieren von Substanzen auf die Oberfläche oder in das Innere dieses mikroskopischen Testobjektes. Ferner umfasst das erfindungsgemäße Kontaktieren auch die Entnahme von Substanzen aus einem derartigen mikroskopischen Testobjekt.

Unter einem "mikroskopischen Testobjekt" wird im Rahmen der vorliegenden Erfindung ein in Suspension befindliches Testobjekt verstanden, das Abmaße im Bereich von wenigen µm bis hin zu mehreren zig µm, vorzugsweise von ca. 5 bis ca.50 µm aufweist. Zu diesen Testobjekten zählen einzelne Zellkulturzellen, Zellcluster, Oozyten, Mikrosphären aus Polymeren, leere Zellhüllen etc.

Diese mikroskopischen Testobjekte werden in der Regel an die erste Öffnung angesaugt und dort durch Unterdruck vorübergehend festgehalten, also immobilisiert. Danach werden sie über die zweite Öffnung kontaktiert, um Messungen an dem Testobjekt vornehmen zu können oder um das Testobjekt im obigen Sinne manipulieren zu können.

In diesem Zusammenhang wird unter einer "Mikrosystemkomponente" ein Bauteil verstanden, das dazu geeignet ist, die mikroskopischen Testobjekte zu immobilisieren oder zu kontaktieren. Derartige Bauteile können als Mikrokanüle oder Pipette ausgebildet sein, sie können auch Elemente aus der Mikrosystemtechnik umfassen, die im Mikrometerbereich positioniert werden können.

"Mikrosystemtechnik" meint miniaturisierte Geräte, Baugruppen und Bauteile, deren Komponenten kleinste Abmessungen im Mikrometerbereich aufweisen, und die als System zusammenwirken.

Die erste Öffnung, an der das mikroskopische Testobjekt zumindest vorübergehend immobilisiert werden soll, kann beispielsweise eine Öffnung an einer Haltepipette oder eine Membranpore an einem mikrosystemtechnischen Bauteil sein. Diese Öffnung kann mit einem in der ersten Mikrosystemkomponente verlaufenden Kanal verbunden sein oder aber als reine Pore ausgebildet sein.

Um das so an der ersten Öffnung immobilisierte Testobjekt kontaktieren zu können, muss die Öffnung einer zweiten Mikrosystemkomponente im Mikrometerbereich zu der ersten Öffnung bzw. zu dem Testobjekt positioniert werden. Diese zweite Öffnung kann an einer Mikrokanüle ausgebildet sein, in der eine Messelektrode angeordnet ist, die Mikrokanüle kann aber auch als Injektionskanüle oder als Absaugkanüle ausgebildet sein.

Aufgrund der Herstellungsprozesse sind bei den hier in Rede stehenden Mikrosystemkomponenten die genauen Positionen der Öffnungen an den Mikrosystemkomponenten jedoch in vielen Fällen nicht bekannt, zumindest nicht in dem hier interessierenden Größenordnungsbereich von wenigen Mikrometern.

Vor diesem Hintergrund sind bisher keine schnellen, preiswerten und zuverlässigen Verfahren bekannt, mit denen zwei derartige Mikrosystemkomponenten automatisiert so zueinander positioniert werden können, dass ihre Öffnungen zueinander ausgerichtet sind.

Im Rahmen der vorliegenden Erfindung wird unter der Angabe "zueinander ausgerichtet" verstanden, dass die beiden Öffnungen einander derart gegenüberliegen, dass ihre Mittenachsen in der jeweils anderen Öffnung verlaufen, vorzugsweise zusammenfallen, und zwischen den Öffnungen ein Abstand herrscht, der nicht mehr als einige Mikrometer beträgt.

Die bisher verfügbaren Verfahren beruhen alle entweder darauf, dass die Position der Öffnung an der jeweiligen Mikrosystemkomponente zuvor von einem Anwender bestimmt wird oder aber im Rahmen eines aufwändigen Bilderkennungsverfahrens bestimmt wird.

Wenn die Position oder Lage der beiden Öffnungen zueinander bekannt ist, lassen sich die Mikrosystemkomponenten dann relativ zueinander so positionieren, dass die Öffnungen zueinander ausgerichtet sind.

Ein erfindungsgemäßes Verfahren sowie eine erfindungsgemäße Vorrichtung sollen dazu verwendet werden, in Suspension befindliche mikroskopische Testobjekte an die erste Öffnung anzusaugen und dort vorübergehend durch Unterdruck festzuhalten. Die zweite Mikrosystemkomponente, die in der Regel als Mikrokanüle ausgebildet ist, wird dann so positioniert, dass die zweite Öffnung auf der Oberfläche oder in einem definierten Abstand zu der Oberfläche des mikroskopischen Testobjektes zu Liegen kommt, oder in das Testobjekt selbst eindringt.

Auf diese Weise können elektrische Messungen an der Oberfläche oder im Inneren des mikroskopischen Testobjektes durchgeführt werden, wobei es auch möglich ist, Substanzen in das Testobjekt einzubringen bzw. aus dem Testobjekt abzusaugen.

Wenn die zweite Öffnung in einem bestimmten Abstand zu dem Testobjekt angeordnet ist, lassen sich die Testobjekte auch transient reizen, indem beispielsweise spezielle Flüssigkeiten für sehr kurze Zeitspannen direkt auf die Oberfläche der Testobjekte gerichtet werden.

Nachdem das Testobjekt so gereizt oder angeregt wurde, kann eine andere Mikrokanüle gegenüber dem Testobjekt positioniert werden, um die Auswirkungen des Reizes zu messen.

Als Testobjekte kommen neben biologischen Zellen oder künstlich hergestellten Mikrosphären auch Microcarrier oder sonstige Festkörper in Frage, die im genannten Durchmesserbereich liegen.

Von besonderem Interesse sind die erfindungsgemäß zu schaffende Vorrichtung sowie das erfindungsgemäß bereitzustellende Verfahren für die Anwendung im Bereich der Patch-Clamp-Technik, deren Entwicklungshistorie und Anwendungsmöglichkeiten beispielsweise in der EP 0 980 523 B1 beschrieben wird.

Bei der konventionell angewandten Patch-Clamp-Technik wird eine Einzelpipette unter mikroskopischer Beobachtung an die Außenseite einer immobilisierten Zelle herangeführt. Dazu wird die fragile Glaspipette mittels eines Mikromanipulators unter optischer Kontrolle von einem Fachmann auf die Zellmembran aufgesetzt, die dann durch Unterdruck an die Öffnung in der Glaspipette angesaugt wird.

Jetzt können entweder Messungen an dem Membranfleck durchgeführt werden, der über die Pipette gegenüber der umgebenden Flüssigkeit abgedichtet und über einen sogenannten Gigaseal elektrisch isoliert ist. Es ist jedoch auch möglich, den Membranfleck durch einen weiteren Druckstoß zu öffnen, so dass unmittelbar Kontakt zu dem Inneren der Zelle besteht.

Ferner ist es auch bekannt, dünn ausgezogene Glaskapillaren in eine Zelle einzustechen und dann Substanzen in das Innere der Zelle zu injizieren bzw. aus dem Inneren der Zelle abzusaugen.

Insbesondere bei den Verfahren, die auf ausgezogenen Patch-Pipetten oder Injektionspipetten beruhen, ist der genaue Ort der Lage der Öffnung an der Pipette nicht bekannt.

Gleiches gilt für die Position von Mikroporen an Oberflächen, auch dort ist die genaue Lage der Mikropore an der Oberfläche häufig produktionsbedingt nicht bekannt.

Wie bereits erwähnt, ist es für die Anwendung der erfindungsgemäß vorgesehenen Techniken jedoch erforderlich, die genaue relative Position zwischen den beiden Öffnungen an zwei Mikrosystemkomponenten zu kennen, die für die Immobilisierung und Kontaktierung eines mikroskopischen Testobjektes vorgesehen sind.

Die bereits erwähnte EP 0 980 523 B1 schlägt in diesem Zusammenhang vor, die Position einer zu kontaktierenden Zelle sowie die Position der Spitze einer Patch-Pipette im Raum mit Hilfe eines bildverarbeitenden Systems automatisiert zu erkennen und dann die Spitze gezielt an die Membranoberfläche der zu kontaktierenden Zelle heranzufahren.

Mit anderen Worten, die Suche nach einer Zelle, mit der eine Patch-Clamp-Verbindung hergestellt werden soll, und die Bestimmung der Lage der Spitze an der Pipette basieren auf optischen Informationen in digitalen Bildern. Das Erkennungsverfahren erfolgt iterativ. Nach der Ermittlung einer ersten groben Abschätzung der Position der Spitze der Pipette und der Positionierung der Pipette in dem gewünschten Teil des Bildes wird die geometrische Mittellinie der Pipette ermittelt, anhand derer dann auf die Position der Öffnung des Kanals in der Pipette geschlossen werden kann.

Dieses Verfahren ist nicht nur extrem zeitaufwändig, es erfordert auch sehr teure und aufwändige Vorrichtungen, die zudem nur von sehr gut ausgebildetem Personal bedient werden können.

Aus diesen Gründen hat das bekannte Verfahren bisher keine breite Anwendung gefunden.

Aus der US 7,384,733 B1 ist es bekannt, eine in Suspension befindliche Zelle an die Spitze einer Patch-Clamp-Pipette anzusaugen. Dazu wird eine Suspension mit zu kontaktierenden Zellen in einer Glaskapillare aufgenommen und dann eine Patch-Clamp-Pipette mittig zu der Öffnung der Glaskapillare angeordnet. Danach wird die Patch-Clamp-Pipette schrittweise auf die Öffnung der Kapillare zu bewegt und der Widerstand der Pipette gegenüber der Umgebung gemessen. Anhand der Widerstandsänderung wird erkannt, wann die Patch-Clamp-Pipette in die Flüssigkeit eintaucht bzw. eine Zelle kontaktiert, die dann durch Unterdruck angesaugt wird.

Bei diesem Verfahren ist zum einen von Nachteil, dass die in Suspension befindlichen Zellen nur durch Zufallstreffer mit der Spitze der Patch-Clamp-Pipette in Kontakt gelangen. Um die Wahrscheinlichkeit zu erhöhen, werden daher viele Zellen in Suspension genommen, auch wenn nur eine dieser Zellen kontaktiert werden soll.

Bei diesem Verfahren ist daher zum einen von Nachteil, dass eine große Anzahl von Zellen vorrätig gehalten werden muss, auch wenn nur jeweils eine Zelle kontaktiert werden soll.

Weiter ist von Nachteil, dass das Verfahren erst dann gestartet werden kann, wenn zuvor die Spitze der Patch-Clamp-Pipette durch Bedienungspersonal koaxial zu der Kapillare ausgerichtet wurde, in der die Zellen in Suspension gehalten werden. Diese Art der Ausrichtung ist jedoch für viele Anwendungsfälle nicht hinreichend präzise. Ferner hängt die Ausrichtung von der Erfahrung des Bedienungspersonals ab und ist damit auch nicht hinreichend zuverlässig und reproduzierbar.

Das bekannte Verfahren setzt überhaupt erst dann an, wenn die Informationen bereits vorliegen, die erfindungsgemäß erst beschafft werden sollen, wobei die Qualität und Reproduzierbarkeit dieser Informationen im Stand der Technik vielfach nicht ausreichend sind.

Das aus der US 7,384,733 B1 bekannte Verfahren ist also nicht vollständig automatisierbar, die Positionierung der Patch-Clamp-Pipette zu der Kapillare muss von Hand erfolgen, was mit den geschilderten Nachteilen verbunden ist.

Ein ähnliches Verfahren beschreibt auch die US 2003/0138767 A1. Nachdem eine Pipette in einen Halter eingeklemmt und zu einem Gefäß ausgerichtet wurde, in dem Zellen in Suspension gehalten werden, wird die Pipette automatisch auf die in dem Gefäß vorhandene Flüssigkeit zu bewegt.

Die Pipette ist eine Patch-Clamp-Pipette, die im Current-Clamp-Modus betrieben wird, in dem der Strom konstant gehalten wird und die sich einstellende Spannung gemessen wird.

Wenn die Pipette in die Lösung eintaucht, wird dies anhand einer Änderung der gemessenen Spannung erkannt.

Danach wird die Patch-Clamp-Pipette auf den Voltage-Clamp-Modus umgestellt, um den Widerstand vor der Pipette zu messen. Anhand einer Widerstandsänderung wird dann erkannt, ob eine Zelle an die Spitze der Pipette angesaugt wurde.

Ein Beispiel für eine Glaspipette oder Glaskapillare, die für Patch-Clamp-Experimente verwendet werden kann, findet sich in der WO 2007/107375 A2.

Das Problem der Positionierung einer Zelle an einer Öffnung, an der sie dann im Sinne der Patch-Clamp-Technik automatisiert kontaktiert werden kann, wird auch in der WO 01/94939 A1 sowie der EP 1 311 655 B1 behandelt.

Diese Verfahren beruhen darauf, dass in Mikrokanalsystemen vereinzelte Zellen durch Kanäle geleitet und an seitlichen Öffnungen in diesen Kanälen angesaugt werden. Die so positionierten Zellen werden dann mittels einer konzentrisch zu der Ansaugöffnung angeordneten Patch-Clamp-Pipette kontaktiert oder über geeignete Pipetten penetriert.

Auch bei diesen Verfahren ist die genaue geometrische Form und Anordnung der Patch-Clamp-Pipette bekannt, bevor das Verfahren überhaupt beginnen kann.

Allen insoweit beschriebenen Verfahren ist gemeinsam, dass aus einer großen Menge von Zellen rein zufällig eine Zelle ausgewählt wird, die anschließend vermessen wird. Dabei kommt es in der Praxis regelmäßig vor, dass tote Zellen, Zelltrümmer oder Verunreinigungen an die Patch-Clamp-Pipette angesaugt werden, was zu einem Abbruch des Experimentes führt. Neben dem Zeitaspekt ist hier weiter von Nachteil, dass die Patch-Clamp-Pipette nicht erneut verwendet werden kann sondern verworfen werden muss.

Es wäre daher wünschenswert, wenn einzelne Zellen gezielt aus einer Menge von verfügbaren Zellen ausgewählt und vor der eigentlichen Messung auf ihre Eignung für die geplante Messung hin überprüft werden könnte. Hierzu sind aber exakte, reproduzierbar beschaffbare Kenntnisse über die Form und Position der Patch-Clamp-Pipette erforderlich,

Vor dem Hintergrund dieses Standes der Technik liegt der vorliegenden Erfindung die Aufgabe zugrunde, ein Verfahren sowie eine Vorrichtung der eingangs genannten Art bereitzustellen, die es ermöglichen, die beiden Mikrosystemkomponenten so zueinander zu positionieren, dass ihre Öffnungen zueinander ausgerichtet sind. Das neue Verfahren soll dabei schnell und zuverlässig auch von weniger hoch spezialisiertem Personal durchgeführt werden können, wobei die neue Vorrichtung einfach und kostengünstig aufgebaut sein soll.

Bei dem eingangs genannten Verfahren wird diese Aufgabe erfindungsgemäß gelöst durch die Schritte:
- unter der Kontrolle einer rechnergesteuerten Steuervorrichtung werden die erste und die zweite Mikrosystemkomponente relativ zueinander im Raum in unterschiedlich relative Positionen zueinander verfahren,
- an einer der beiden Mikrosystemkomponenten werden elektrische Signale abgegeben, die an der anderen der beiden Mikrosystemkomponenten als Messwerte aufgenommen werden, die von der relativen Position der beiden Mikrosystemkomponenten zueinander abhängen,
- für unterschiedliche relative Positionen zwischen den beiden Mikrosystemkomponenten werden Messwerte bestimmt, aus denen in der Steuervorrichtung ermittelt wird, in welcher relativen Position die beiden Mikrosystemkomponenten so zueinander zu positionieren sind, dass ihre Öffnungen zueinander ausgerichtet sind.

Bei der erfindungsgemäß verwendeten, eingangs genannten Vorrichtung sind zur Durchführung des neuen Verfahrens eine Mechanik, über die die beiden Mikrosystemkomponenten relativ zueinander zumindest in zwei zueinander orthogonalen Raumachsen verfahrbar sind, eine Steuervorrichtung, über die die Mechanik automatisiert gesteuert wird, und eine Messanordnung vorgesehen, über die an einer der beiden Mikrosystemkomponenten elektrische Signale abgegeben werden, die an der anderen der beiden Mikrosystemkomponenten als Messwerte aufgenommen werden, die von der relativen Position der beiden Mikrosystemkomponenten zueinander abhängen, wobei die Steuervorrichtung dazu eingerichtet ist, aus Messwerten für unterschiedliche relative Positionen zwischen den beiden Mikrosystemkomponenten zu ermitteln, in welcher relativen Position die beiden Mikrosystemkomponenten so zueinander zu positionieren sind, dass ihre Öffnungen zueinander ausgerichtet sind.

Die der vorliegenden Erfindung zugrunde liegende Aufgabe wird auf diese Weise vollkommen gelöst.

Die vorliegende Erfindung schafft damit erstmals die Grundlage für ein einfaches und zuverlässiges automatisiertes Verfahren sowie eine preiswerte automatisierte Vorrichtung, mit denen mikroskopische Testobjekte, vorzugsweise biologische Zellen, auf schnelle und reproduzierbare Weise gezielt immobilisiert und kontaktiert werden können.

Die Erfinder der vorliegenden Anmeldung haben nämlich erkannt, dass es auch im Mikrometerbereich möglich ist, Mikrosystemkomponenten in unterschiedliche relative Positionen zueinander zu verfahren und für diese jeweiligen Positionen Messwerte aufzunehmen, die ein Maß für die relative Position zueinander sind. Wenn eine größere Menge solcher Messwerte aufgenommen wurde, kann dann automatisiert berechnet werden, bestimmt werden, wie die Mikrosystemkomponenten zueinander positioniert werden müssen, damit ihre jeweiligen Öffnungen zueinander ausgerichtet sind, ihre jeweiligen Mittellinien in die jeweils andere Öffnung hinein verlaufen und der Abstand zwischen den Öffnungen nur einige Mikrometer beträgt, so dass ein flächiger kontakt gerade noch vermieden ist.

Das neue Verfahren verzichtet dabei völlig auf optische oder bildverarbeitende Vorrichtungen und Maßnahmen, wobei auch der Eingriff von Bedienungspersonen nicht erforderlich ist.

Vielmehr müssen lediglich die beiden Mikrosystemkomponenten an der Mechanik montiert und in das entsprechende flüssige Medium eingebracht werden, woraufhin dann das "Suchverfahren" beginnen kann. Die Suchstrategie ist dabei so programmiert, dass sowohl für die Durchführung als auch für die Auswertung des Suchverfahrens manuelle Eingriffe nicht erforderlich sind.

Nicht erwartet werden konnte dabei, dass dieses auf der Übertragung von elektrischen Signalen beruhende Verfahren es ermöglicht, die relative Position von mikroskopischen Öffnungen in Mikrosystemkomponenten mit der erforderlichen Genauigkeit zu bestimmen.

In diesem Zusammenhang sei daran erinnert, dass die Größe der Öffnungen in den Mikrosystemkomponenten im Bereich <1 µm bis höchstens 10 µm liegt, so dass Testobjekte positioniert werden können, deren Durchmesser vorzugsweise im Bereich von 5 bis 50 µm liegen.

Die geometrische Form der Mikrosystemkomponenten ist zudem nicht vorgegeben und reproduzierbar, vielmehr sind insbesondere die ausgezogenen Spitzen von Glaskapillaren sowohl unterschiedlich lang als auch in ihrer Form unterschiedlich, sie können stärker oder weniger gebogen oder gekrümmt sein. Ferner ist zu bedenken, dass die Öffnungen nicht immer mittig an den Mikrosystemkomponenten angeordnet sind, sie liegen vielmehr fertigungsbedingt sehr unterschiedlich in der Austrittsfläche der jeweiligen Mikrosystemkomponenten.

Dies gilt auch für Mikroporen an Oberflächen von Mikrosystemkomponenten, insbesondere aber für solche Öffnungen, die als Mündung eines Kanals dienen, der in der jeweiligen Mikrosystemkomponente vorgesehen ist.

Durch diese Kanäle können im Fall einer zum Halten eines Testobjektes vorgesehenen Mikrosystemkomponente Unterdrücke appliziert werden, durch die das Testobjekt an die Öffnung angesaugt und dann auf der Öffnung immobilisiert wird. Ferner kann durch diesen Kanal auch ein Überdruck appliziert werden, um das immobilisierte Testobjekt wieder von der Öffnung zu entfernen.

Handelt es sich bei der Mikrosystemkomponente dagegen beispielsweise um eine Pipette oder Kanüle zum Kontaktieren eines immobilisierten Testobjektes, so kann in dem Kanal eine Elektrode vorgesehen sein, um Messungen an oder in dem Testobjekt durchführen zu können. Ferner können durch den Kanal Testsubstanzen auf das Testobjekt oder in das Testobjekt geleitet bzw. Substanzen aus dem Testobjekt abgesaugt werden.

Für all diese unterschiedlichen Funktionen sind Mikrosystemkomponenten, insbesondere Patch-Clamp-Pipetten oder Glaskapillare, vorgesehen, für die sich unabhängig von ihrer geometrischen Form mit Hilfe der neuen Vorrichtung bzw. des neuen Verfahrens der Ort der Öffnung an der Spitze in Relation zu dem Ort der Öffnung an der Spitze der anderen Mikrosystemkomponente genau bestimmen lässt.

Mit anderen Worten, das Ergebnis des neuen Suchverfahrens ist die Angabe der relativen Position zwischen den beiden Mikrosystemkomponenten, in der die Öffnungen im beschriebenen Maß zueinander ausgerichtet sind. Da bei beiden Mikrosystemkomponenten die genaue Position der Öffnung an der Mikrosystemkomponente selbst nicht bekannt ist, liefert das neue Verfahren keine exakte Information darüber, wo die Position der Öffnung an der jeweiligen Mikrosystemkomponente ist, vielmehr liefert es eine Information darüber, wie das Gesamtsystem aus Mechanik und Mikrosystemkomponenten relativ zu positionieren ist, damit die Öffnungen zueinander ausgerichtet sind.

Dabei ist es selbstverständlich nicht erforderlich, beide Mikrosystemkomponenten im Raum zu verfahren, es reicht völlig aus, wenn eine der beiden Mikrosystemkomponenten verfahren wird.

In einer Weiterbildung ist es dann bevorzugt, wenn die elektrischen Signale über eine in dem ersten Kanal angeordnete Signalelektrode in das Medium abgegeben und die Messwerte über eine in dem zweiten Kanal angeordnete Messelektrode aufgenommen werden, wobei vorzugsweise die elektrischen Signale in das Medium abgegebene Spannungsimpulse sind, deren Pulsamplituden an der zweiten Mikrosystemkomponente als Messwerte erfasst werden.

Hier ist von Vorteil, dass eine besonders einfache und dennoch besonders genaue Art, die für die relative Position kennzeichnenden Messwerte zu bestimmen, darin besteht, an den Öffnungen selbst Signale in das Medium abzugeben bzw. aus dem Medium aufzunehmen und als Messwert weiterzuverarbeiten.

Bei dieser Maßnahme ist besonders überraschend, dass einfache Spannungsimpulse als elektrische Signale verwendet werden können, wobei deren Pulsamplitude ein Maß für die relative Position der Mikrosystemkomponenten, genauer der Öffnungen an den Mikrosystemkomponenten zueinander, ist.

Dabei ist es weiter bevorzugt, wenn die beiden Mikrosystemkomponenten in zumindest zwei zueinander orthogonalen Raumachsen zueinander verfahren werden, wobei die erste Raumachse etwa parallel zu den Projektionen der Längsachsen der beiden Öffnungen in die durch die beiden Raumachsen aufgespannte Ebene verläuft.

Bei dieser Maßnahme ist von Vorteil, dass das Suchverfahren relativ einfach durchgeführt wird. Zunächst werden die beiden Mikrosystemkomponenten zueinander in der ersten Raumachse verfahren, wobei für verschiedene Positionen entlang dieser Raumachse die Messwerte genommen werden. Danach werden die Mikrosystemkomponenten in der zweiten Raumachse zueinander verfahren, wobei auch hier für bestimmte Positionen längs der zweiten Raumachse die Messwerte genommen werden.

Aus dem Verlauf der Messwerte längs der ersten und der zweiten Raumachse lässt sich dann die relative Position für die beiden Mikrosystemkomponenten zueinander bestimmen, in der die Öffnungen zueinander ausgerichtet sind.

Die erste Raumachse läuft dabei etwa parallel zu den Projektionen der Längsachsen der Mikrosystemkomponenten in die Ebene, die durch die beiden Raumachsen aufgespannt wird. Dies bedeutet nichts anderes, als dass die erste Raumachse etwa parallel zu den Mikrosystemkomponenten verläuft, unabhängig davon, ob die Mikrosystemkomponente selbst in der durch die beiden Raumachsen aufgespannten Ebene liegt oder von schräg oben in diese Ebene "eintaucht". Diese schräge Anordnung kann von Vorteil sein, wenn die Mikrosystemkomponenten seitlich von oben in das Medium eingetaucht werden, das zum Transport der elektrischen Signale, vor allem aber zur Aufnahme der in Suspension gehaltenen Testobjekte dient.

Durch diese Art der Anordnung wird vermieden, dass es während der Durchführung des neuen Verfahrens zu einem Crash zwischen den Spitzen der beiden Mikrosystemkomponenten kommen kann. Wenn die Mikrosystemkomponenten zuerst längs der ersten Raumachse zueinander verfahren werden, laufen sie parallel nebeneinander her, wobei die Messwerte dann nach der Erfahrung der Erfinder bereits angeben, auf welcher "Höhe" in der ersten Raumachse die beiden Öffnungen etwa liegen. Das Steuerungssystem wird dann die beiden Mikrosystemkomponenten in der ersten Raumachse zueinander so anordnen, dass ein Sicherheitsabstand zwischen den Öffnungen in Richtung der ersten Raumachse ist. In dieser Position wird dann das System die beiden Mikrosystemkomponenten in der zweiten Raumachse zueinander verfahren, wobei hier dann kein Crash auftreten kann, weil der Sicherheitsabstand zwischen den Spitzen eingestellt wurde.

Dabei ist es bevorzugt, wenn in der ersten Raumachse eine grobe relative Position bestimmt wird, indem bei gleichbleibender fester relativer Position in der zweiten Raumachse die beiden Mikrosystemkomponenten in der ersten Raumachse in verschiedene relative Positionen gefahren werden und für jede relative Position ein Messwert bestimmt wird, wobei vorzugsweise in der zweiten Raumachse eine grobe relative Position bestimmt wird, indem bei gleichbleibender fester relativer Position in der ersten Raumachse die beiden Mikrosystemkomponenten in der zweiten Raumachse in verschiedene relative Positionen gefahren werden und für jede relative Position ein Messwert genommen wird.

Die feste relative Position in der zweiten Raumachse wird dabei so gewählt, dass zwischen den beiden Mikrosystemkomponenten in Richtung der zweiten Raumachse ein Sicherheitsabstand besteht, der in Abhängigkeit von den Abmaßen der Mikrosystemkomponenten in Richtung der zweiten Raumachse bestimmt wird. Vorzugsweise wird dann die feste relative Position in der ersten Raumachse so gewählt, dass zwischen den beiden Öffnungen in Richtung der ersten Raumachse ein Sicherheitsabstand besteht, der in Abhängigkeit von der zuvor bestimmten, groben relativen Position in der ersten Raumachse bestimmt wird.

Sowohl die Starteinstellung als auch die Durchführung dieses Verfahrens benötigt keinen Input von Bedienungspersonal, denn in der Startstellung wird die Mechanik automatisch so eingestellt, dass bei eingespannter erster Mikrosystemkomponente und eingespannter zweiter Mikrosystemkomponente diese in Richtung der zweiten Raumachse eine relative Startposition aufweisen, die einen hinreichenden Sicherheitsabstand gewährleistet. In Abhängigkeit von der Art der Mikrosystemkomponenten bzw. in Abhängigkeit von deren Abmaßen wird dabei dieser Sicherheitsabstand entweder anhand eines Algorithmus berechnet oder aber aus einer entsprechenden Tabelle entnommen.

Für den Fall, dass die neue Vorrichtung und das neue Verfahren in einer speziellen Laborsituation immer mit speziellen Haltepipetten und Patch-Clamp-Pipetten von bekannten makroskopischen geometrischen Dimensionen verwendet werden, kann auch immer die gleiche Startposition mit entsprechendem Sicherheitsabstand verwendet werden.

Die insoweit beschriebenen Verfahrensschritte führen dann dazu, dass sowohl in der ersten Raumachse als auch in der zweiten Raumachse eine relative grob aufgelöste Position bestimmt wird, in der die Öffnungen zueinander bereits grob ausgerichtet sind.

Um diese Ausrichtung jetzt zu verfeinern, die Öffnungen also noch genauer zueinander auszurichten, wird in der ersten Raumachse eine fein aufgelöste relative Position bestimmt, indem bei gleichbleibender grober relativer Position in der zweiten Raumachse die beiden Mikrosystemkomponenten in der ersten Raumachse in verschiedene relative Positionen gefahren und für jede relative Position ein Messwert genommen wird, wobei ferner vorzugsweise in der zweiten Raumachse eine fein aufgelöste relative Position bestimmt wird, indem bei gleichbleibender, fein aufgelöster relativer Position in der ersten Raumachse die beiden Mikrosystemkomponenten in der zweiten Raumachse in verschiedene relative Positionen gefahren werden und für jede relative Position ein Messwert genommen wird.

Diese Schritte zur Bestimmung der fein aufgelösten relativen Position sind somit eine Wiederholung der Schritte, die zu der grob aufgelösten relativen Position geführt haben, wobei die Startpositionen in der ersten und der zweiten Raumachse sich jetzt jeweils aus der relativen Position bestimmen, die im jeweils vorhergehenden Verfahrensschritt für die jeweils andere Achse bestimmt wurde.

Mit anderen Worten, das Verfahren geht aus von einem Sicherheitsabstand in der zweiten Raumachse und bestimmt die grobe relative Position in der ersten Raumachse. Ausgehend von dieser groben relativen Position in der ersten Raumachse wird dann eine grobe relative Position in der zweiten Raumachse bestimmt. Ausgehend von dieser relativen groben Position in der zweiten Raumachse wird dann eine fein aufgelöste relative Position in der ersten Raumachse bestimmt, die dann wiederum Ausgangspunkt für die Bestimmung der fein aufgelösten relativen Position in der zweiten Raumachse ist.

Diese Verfahrensschritte können mehrfach nacheinander wiederholt werden, um so letztendlich zu einer relativen Position zwischen den beiden Mikrosystemkomponenten in der ersten und zweiten Raumachse zu gelangen, in der die beiden Öffnungen hinreichend genau zueinander ausgerichtet sind.

Die Verfahrensschritte können beispielsweise mehrfach iterativ so lange wiederholt werden, bis sich keine Verbesserungen in den relativen Positionen mehr ergeben, bzw. bis die Verbesserungen im Mikrometerbereich liegen.

Selbstverständlich ist es möglich, auf genau die gleiche Weise auch die relative Position der beiden Mikrosystemkomponenten zueinander in einer dritten Raumachse zu bestimmen, die orthogonal zu der ersten und der zweiten Raumachse liegt. Ausgangspunkt für die Durchführung des Verfahrens in der dritten Raumachse kann dabei entweder eine Sicherheitsposition, eine grobe relative Position oder aber eine fein aufgelöste relative Position sein.

Dabei ist es bevorzugt, wenn die beiden Mikrosystemkomponenten in einer dritten Raumachse relativ zueinander verfahren werden, die orthogonal zu der ersten und zweiten Raumachse verläuft, wobei in der dritten Raumachse eine relative Position bestimmt wird, indem bei fester relativer Position in der ersten und zweiten Raumachse die beiden Mikrosystemkomponenten in der dritten Raumachse in verschiedene relative Positionen gefahren und für jede relative Position ein Messwert genommen wird.

Allgemein ist es noch bevorzugt, wenn die erste Mikrosystemkomponente zum Immobilisieren einer biologischen Zelle und die zweite Mikrosystemkomponente zum Kontaktieren der so immobilisierten Zelle ausgerüstet ist.

Bei dieser Maßnahme ist von Vorteil, dass das neue Verfahren sowie die neue Vorrichtung dann im Zusammenhang mit Untersuchungen nach der Patch-Clamp-Technik oder zum Injizieren von Substanzen in die Zelle bzw. zum Entnehmen von Substanzen aus der Zelle verwendet werden können.

Demgemäß betrifft die vorliegende Erfindung auch ein Verfahren zum automatisierten Kontaktieren von zumindest einem mikroskopischen Testobjekt, das an einer Öffnung einer ersten Mikrosystemkomponente immobilisiert und dann über eine Öffnung an einer zweiten Mikrosystemkomponente kontaktiert wird, bei dem
- mit dem neuen Verfahren die relative Position zwischen den beiden Öffnungen bestimmt wird, dann
- an der ersten Öffnung ein Testobjekt immobilisiert wird, dann
- vorzugsweise das Testobjekt auf seine Eignung für nachfolgende Untersuchungen überprüft wird, dann
- die beiden Öffnungen längs einer ersten Raumrichtung zueinander mit einem Abstand ausgerichtet werden, der mindestens dem doppelten maximalen Durchmesser des zu kontaktierenden Testobjektes entspricht, dann
- mittels einer Sensorelektrode der Widerstand vor der zweiten Öffnung gemessen wird, und dann
- in einem Suchverfahren der Abstand zwischen den beiden Öffnungen in der ersten Richtung schrittweise reduziert und für jeden neuen Abstand erneut der Widerstand gemessen wird, wobei
- bei einer vorgegebenen Änderung des gemessenen Widerstandswertes das Suchverfahren beendet und das Testobjekt als kontaktiert bewertet wird.

Dieses Verfahren geht deutlich über das Verfahren hinaus, wie es aus den eingangs diskutierten Dokumenten US 7,384,733 B1 und US 2003/0138767 A1 bekannt ist, weil jetzt erfindungsgemäß die Position der Öffnungen zueinander mit dem neuen Verfahren bestimmt wird.

Sobald diese relative Position bekannt ist, können die beiden Mikrosystemkomponenten relativ zueinander so verfahren werden, dass ihre Öffnungen in der ersten Raumachse einen bestimmten Abstand zueinander aufweisen, in der zweiten und ggf. dritten Raumachse jedoch zueinander ausgerichtet sind.

Dieser Abstand zwischen den beiden Öffnungen in der ersten Raumachse wird dabei so ausgewählt, dass er deutlich größer ist als der Durchmesser eines zu immobilisierenden Testobjektes.

Die Umgebung der beiden Mikrosystemkomponenten wird dann mit einer Lösung gespült, in der sich Testobjekte in Suspension befinden. An der Öffnung der ersten Mikrosystemkomponente wird dabei ein geringer Unterdruck angelegt, der dazu führt, dass vorbei diffundierende Testobjekte an die erste Öffnung angesaugt werden. Anhand einer Veränderung des Unterdrucks oder anhand einer Veränderung des elektrischen Feldes an oder vor der ersten Öffnung wird dabei erkannt, dass eine Zelle angesaugt und immobilisiert wurde.

Danach wird dann gemäß dem beschriebenen Verfahren die zweite Mikrosystemkomponente auf die erste Mikrosystemkomponente schrittweise zu bewegt und dabei der Widerstand im Voltage-Clamp-Modus und/oder Current-Clamp-Modus kontinuierlich gemessen. Anhand einer Änderung des Widerstandes kann dann erkannt werden, dass die zweite Öffnung die Membran des Testobjektes berührt hat.

Aus den relativen Positionen der Mikrosystemkomponenten zueinander bei unmittelbar voreinander liegenden Öffnungen einerseits und bei einem immobilisierten Testobjekt andererseits lässt sich jetzt der Durchmesser des Testobjektes bestimmen, er entspricht der Differenz der relativen Positionen in Richtung der ersten Raumachse.

Die Information über den Durchmesser des Testobjektes kann jetzt zum einen verwendet werden, um zu überprüfen, ob überhaupt ein gewünschtes Testobjekt immobilisiert wurde. Auf diese Weise lässt sich beispielsweise feststellen, ob Zelltrümmer oder defekte Testobjekte immobilisiert wurden oder ob ein für das weitere Verfahren geeignetes Testobjekt "eingefangen" wurde.

Diese Beurteilung über die Eignung des "eingefangenen" Testobjektes für die geplanten Untersuchungen kann auch erfolgen, indem das an der ersten Öffnung immobilisierte Testobjekt optisch, elektrisch oder durch andere geeignete Verfahren untersucht wird. Damit kann aus einer großen Menge von Zellen, die ggf. nur wenige Zellen enthält, die tatsächlich für die geplante Untersuchung geeignet sind, gezielt eine der geeigneten Zellen ausgesucht werden. Ist das immobilisierte Testobjekt nicht geeignet, wird es verworfen und eine neue Zelle angesaugt.

Mit dem neuen Verfahren kann somit zwar nicht gezielt ausschließlich eine geeignete Zelle unmittelbar aus der Suspension herausgefischt werden. Die zufällig immobilisierten Zellen können jedoch auf ihre Eignung untersucht werden, bevor das eigentliche Untersuchungsverfahren beginnt. Damit ist es nicht erforderlich, den Anteil der geeigneten Zellen in der Suspension vor der eigentlichen Messung durch weitere Verfahren zu erhöhen, was zudem nicht immer möglich ist.

In das immobilisierte und qualifizierte Testobjekt kann jetzt die zweite Mikrosystemkomponente beispielsweise eingestochen werden, wenn sie eine Glaskanüle ist, über die Substanzen in das Testobjekt eingebracht oder aus dem Testobjekt entnommen werden können. Andererseits kann die zweite Öffnung so auf die Oberfläche des Testobjektes aufgelegt werden, dass ein so genannter Gigaseal entsteht, so dass Messungen entweder an dem umgrenzenden Membranfleck oder nach entsprechender Perforierung des Membranflecks an dem gesamten Testobjekt vorgenommen werden können.

Von besonderem Vorteil ist dabei, dass das Halten des Testobjektes nicht durch die Patch-Clamp-Pipette sondern durch die erste Öffnung erfolgt, die beispielsweise an einer Haltepipette ausgebildet sein kann. Dadurch wird vermieden, dass beim Immobilisieren der Zelle Flüssigkeit in die Patch-Clamp-Pipette eingesogen wird, die sich dort mit der Flüssigkeit in der Patch-Clamp-Pipette vermischen würde.

Diese Flüssigkeit in der Patch-Clamp-Pipette unterscheidet sich nämlich aus biologischen und messtechnischen Gründen von der Flüssigkeit, in der sich die Zellen in Suspension befinden, so dass im Stand der Technik durch aufwändige maßnahmen verhindert werden muss, dass diese Vermischung sich negativ auswirkt.

Da erfindungsgemäß jetzt die beiden Öffnungen erstmals automatisch zueinander mit hoher Genauigkeit positioniert werden können, kann die Patch-Clamp-Pipette von dem Ansaugen entlastet werden, weil sie nach dem Immobilisieren und ggf. Qualifizieren der Zelle an der ersten Öffnung genau auf der Oberfläche der Zelle positioniert werden kann.

Ferner ist es möglich, über die zweite Mikrosystemkomponente gezielt Testsubstanzen auf die Oberfläche eines immobilisierten Testobjektes zu geben, wobei der Abstand zwischen der Oberfläche des Testobjektes und der zweiten Öffnung anhand der nach dem erfindungsgemäßen Verfahren bestimmten Informationen beliebig, aber mit hinreichender Genauigkeit gewählt werden kann.

Dabei ist es bevorzugt, wenn während des Immobilisierens des Testobjektes an der ersten Öffnung mittels einer Sensorelektrode der Widerstand vor der ersten Öffnung gemessen wird.

Dieses Verfahren wird auf gleiche Weise durchgeführt wie die zuvor beschriebene Erkennung des Kontaktes des bereits immobilisierten Testobjektes an der zweiten Öffnung.

Des weiteren ist es möglich, das an der ersten Öffnung immobilisierte und nach dem beschriebenen Verfahren erfolgreich kontaktierte Testobjekt mit Hilfe der zweiten Mikrosystemkomponente von der ersten Mikrosystemkomponente wegzubewegen, indem die beiden Mikrosystemkomponenten relativ zueinander verfahren werden.

Hierzu kann entweder die natürliche Adhäsion des Testobjektes an der zweiten Mikrosystemkomponente oder ein an der zweiten Mikrosystemkomponente angelegter leichter Unterdruck ausgenutzt werden, um das Testobjekt an der zweiten Mikrosystemkomponente festzuhalten.

Hierdurch ist es möglich, das Testobjekt an einen anderen Ort zu verbringen. Insbesondere ist es möglich, das Testobjekt mit Hilfe der zweiten Mikrosystemkomponente gegenüber der Öffnung der ersten Mikrosystemkomponente in einem geeigneten Abstand zu positionieren, und nunmehr über die erste Mikrosystemkomponente gezielt Testsubstanzen auf die Oberfläche eines immobilisierten Testobjektes zu geben. Der Abstand zwischen der Oberfläche des Testobjektes und der Öffnung kann dabei anhand der nach dem erfindungsgemäßen Verfahren bestimmten Informationen beliebig, aber mit hinreichender Genauigkeit gewählt werden.

Dies ist insbesondere deshalb Vorteilhaft, weil in dieser Konfiguration gleichzeitig mit der zweiten Mikrosystemkomponente eine Messung am Testobjekt durchgeführt werden kann, während mit der ersten Mikrosystemkomponente eine Testsubstanz auf das Testobjekt gegeben wird.

In einer Variante des oben beschriebenen Verfahrens wird eine weitere dritte Mikrosystemkomponente verwendet, um Testsubstanzen auf die Oberfläche des Testobjekts zu geben. Diese dritte Mikrosystemkomponente kann mechanisch fest mit der ersten Mikrosystemkomponente verbunden sein, so dass die nach dem erfindungsgemäßen Verfahren bestimmten Informationen über den Ort der Öffnung an der ersten Mikrosystemkomponente auch eine ausreichende Information über den Ort der Öffnung an der dritten Mikrosystemkomponente darstellen.

Diese Information kann sich entweder aus dem Aufbau ergeben oder aber mit Hilfe einer zweiten Öffnung einmal nach dem erfindungsgemäßen Verfahren bestimmt werden. Auf diese Weise wird zu Beginn einer Messreihe auf erfindungsgemäße Weise einmal die relative Position zwischen erster und zweiter sowie zwischen dritter und zweiter Öffnung bestimmt, und aus diesen Informationen die unveränderliche relative Position zwischen erster und dritter Öffnung bestimmt und für die weiteren Messungen verwendet.

Während die erste und dritte Mikrosystemkomponente während der Messreihe nicht ausgewechselt werden, kann die zweite Mikrosystemkomponente, die eine Patch-Clamp-Pipette sein kann, ausgewechselt werden.

Nach diesem Auswechseln muss lediglich die relative Position zwischen neuer zweiter und bisheriger erster Öffnung erneut bestimmt werden, die relative Position zwischen neuer zweiter und bisheriger dritter Öffnung lässt sich dann aus den vorherigen Messungen ermitteln.

Andererseits kann bei dem erfindungsgemäßen Verfahren auch jedes mal zunächst die relative Position zwischen der ersten und der zweiten Öffnung und dann die relative Position zwischen der zweiten und einer dritten Öffnung an einer dritten Mikrosystemkomponente bestimmt werden.

Allgemein betrifft die vorliegende Erfindung auch eine Vorrichtung, die dazu eingerichtet ist, das neue Verfahren durchzuführen.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der beigefügten Zeichnung dargestellt und werden in der nachfolgenden Beschreibung näher erläutert. Es zeigen:
- Fig. 1: eine schematische Draufsicht auf eine für die Durchführung des neuen Verfahrens verwendete Vorrichtung in ihrer Grundposition;
- Fig. 2: eine Vorderansicht des bei der Vorrichtung aus Fig. 1 eingesetzten Reaktionsgefäßes, in das zwei Mikrosystemkomponenten mit ihren Spitzen eingetaucht sind;
- Fig. 3: in einer schematischen Draufsicht die Spitzen von zwei Mikrosystemkomponenten sowie Diagramme von Messwerten für verschieden relative Positionen der Mikrosystemkomponenten zueinander;
- Fig. 4: in einer Darstellung wie Fig. 3 zwei Mikrosystemkomponenten, deren Öffnungen zueinander ausgerichtet sind;
- Fig. 5: in einer Darstellung wie Fig. 3 zwei Mikrosystemkomponenten, von denen an einer ein mikroskopisches Testobjekt immobilisiert ist, wobei ferner ein Diagramm zur Bestimmung des Durchmessers des Testobjektes gezeigt ist, und
- Fig. 6: eine Variante der in Fig. 1 gezeigten Vorrichtung.

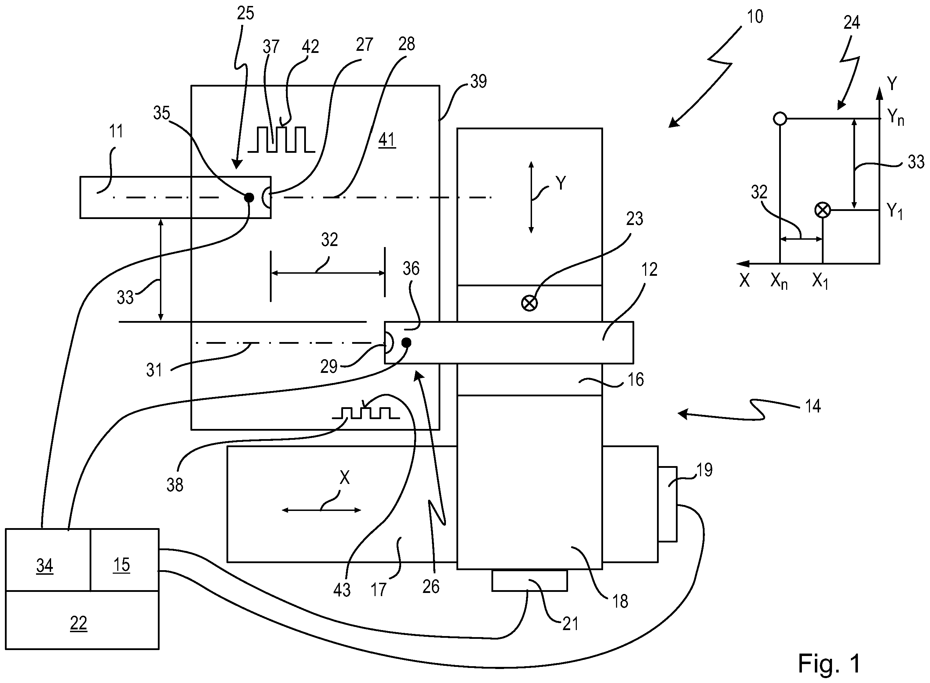

In Fig. 1 ist mit 10 schematisch eine Vorrichtung zur automatisierten Bestimmung der relativen Position zwischen zwei Mikrosystemkomponenten 11 und 12 gezeigt.

Die Vorrichtung 10 umfasst eine Mechanik 14, über die die beiden Mikrosystemkomponenten 11 und 12 relativ zueinander unter der Kontrolle einer rechnergesteuerten Steuervorrichtung 15 in den beiden orthogonalen Raumachsen x und y zueinander verfahren werden können.

Die Mechanik 14 umfasst dazu einen Schlitten 16, an dem die zweite Mikrosystemkomponente 12 befestigt ist. Die erste Mikrosystemkomponente 11 ist raumfest angeordnet.

.Die Mechanik 14 umfasst ferner eine x-Führung 17, an der eine in x-Richtung verfahrbare y-Führung 18 angeordnet ist, auf der in y-Richtung verfahrbar der Schlitten 16 montiert ist.

Die y-Führung wird mittels eines x-Motors 19 in x-Richtung und der Schlitten 16 mittels eines y-Motors 21 in y-Richtung verfahren

Die Bewegungen des Schlittens 16 erfolgen unter der Kontrolle eines Rechners 22, in den die Steuervorrichtung 15 integriert sein kann.

An dem Schlitten 16 ist beispielhaft ein Referenzpunkt 23 angedeutet, dessen Position oder Lage in einem bei 24 angedeuteten Koordinatensystem von dem Rechner 22 vorgegeben wird.

In der in Fig. 1 gezeigten Grundstellung befindet sich der Schlitten 16, also der Referenzpunkt 23, in der Position x1/y1, die in dem Koordinatensystem 24 angedeutet ist.

Die beiden Mikrosystemkomponenten 11 und 12 sollen nun relativ zueinander so verfahren werden, dass ihre Spitzen 25 und 26 zueinander ausgerichtet sind. Genauer gesagt geht es darum, eine Öffnung 27 mit einer Längsachse 28 an der ersten Mikrosystemkomponente 11 zu einer Öffnung 29 mit einer Längsachse 31 an der zweiten Mikrosystemkomponente 12 so zueinander auszurichten, dass sie unmittelbar voreinander liegen, so dass sich die Spitzen 25 und 26 mit ihren Stirnseiten nahezu berühren und die Längsachse 28 in der Öffnung 29 liegt, während die Längsachse 31 in der Öffnung 27 liegt. Vorzugsweise sollen die Längsachsen 28 und 31 zusammenfallen, wenn die Öffnungen 27 und 29 erfindungsgemäß zueinander ausgerichtet sind.

Um diese Ausrichtung der Öffnungen 27 und 29 zu erreichen, muss die Mikrosystemkomponente 12 in x-Richtung um einen Stellweg 32 und in y-Richtung um einen Stellweg 33 verfahren werden, wie dies in Fig. 1 sowohl an der Vorrichtung 10 als auch in dem Koordinatensystem 24 angedeutet ist.

Problematisch ist dabei nun, dass die genaue Position der Spitze 25 bzw. der Öffnung 27 genauso wenig bekannt ist, wie die genaue Position der Öffnung 29 zu dem Referenzpunkt 23.

Diese Unsicherheit liegt an den herstellungsbedingten Schwankungen in der Länge der Spitzen 25 und 26 sowie in der Lage der Öffnungen 27 und 29 an den Spitzen 25 und 26.

Die Stellwege 32 und 33 sind somit nicht bekannt sondern müssen zunächst ermittelt werden, damit die Mikrosystemkomponente 12 in eine relative Position zu der Mikrosystemkomponente 11 verfahren werden kann, in der die Öffnungen 27 und 29 zueinander ausgerichtet sind.

Erfindungsgemäß ist daher in dem Rechnern 22 eine Messanordnung 34 vorgesehen, die mit einer Signalelektrode 35 an der Öffnung 27 in der ersten Mikrosystemkomponente 11 sowie mit einer Messelektrode 36 an der Öffnung 29 in der Mikrosystemkomponente 12 zusammenwirkt.

Über die Messanordnung 34 wird an der Signalelektrode 35 eine zeitliche Folge von Spannungsimpulsen 27 ausgegeben, die als zeitliche folge von Messwerten 38 an der Messelektrode 36 gemessen werden.

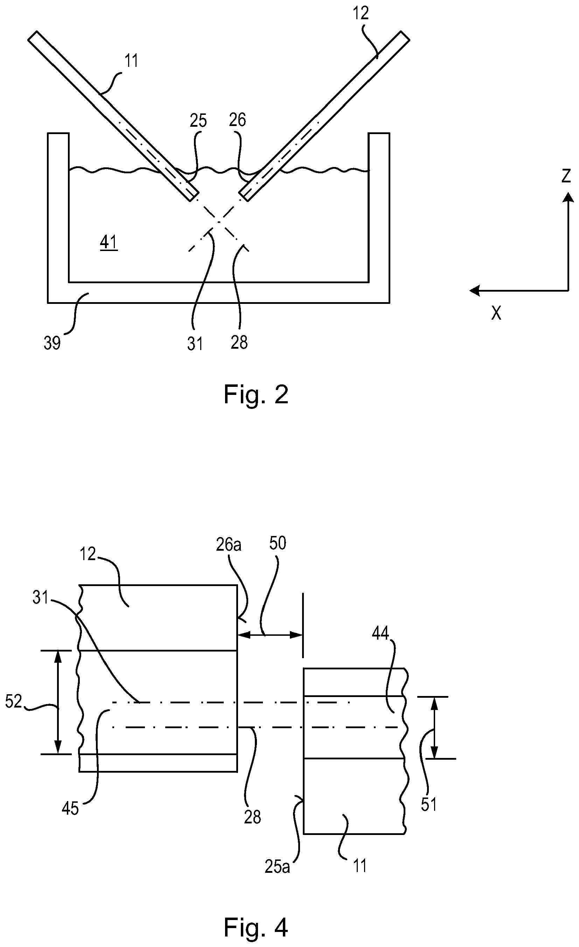

Damit diese Messung möglich ist, sind die Spitzen 25 und 26 der Mikrosystemkomponenten 11 und 12 in ein Reaktionsgefäß 39 eingetaucht, das mit einem flüssigen Medium 41 gefüllt ist, wie dies insbesondere in der Seitenansicht der Fig. 2 zu erkennen ist.

Das Reaktionsgefäß 39 und das Medium 41 werden auch dafür benötigt, um Testobjekte zu den Mikrosystemkomponenten 11, 12 zu führen und an diesen Testobjekten Messungen oder Manipulationen vorzunehmen, wie dies nachstehend noch genauer beschrieben wird.

Aus der Fig. 2 ist erkennbar, dass die Mikrosystemkomponenten 11 und 12 mit ihren Längsachsen 28 und 31 in der x/z-Ebene geneigt angeordnet sind, sie weisen also mit ihren Spitzen 25 und 26 von oben, also aus z-Richtung gesehen, schräg in das Medium 41 hinein.

Die Anordnung ist dabei so getroffen, dass die Projektion der Längsachsen 28 und 31 in der x/y-Ebene, wie es in Fig. 1 gezeigt ist, parallel zu der x-Achse und quer zu der y-Achse verläuft.

Zurückkehrend zu Fig. 1 ist zu erkennen, dass die Spannungsimpulse 37 eine Pulsamplitude 42 aufweisen, die einen höheren Wert aufweist als die Pulsamplituden 43 der Messwerten 38, die an der Messelektrode 36 erfasst und zu der Messanordnung 34 geleitet werden.

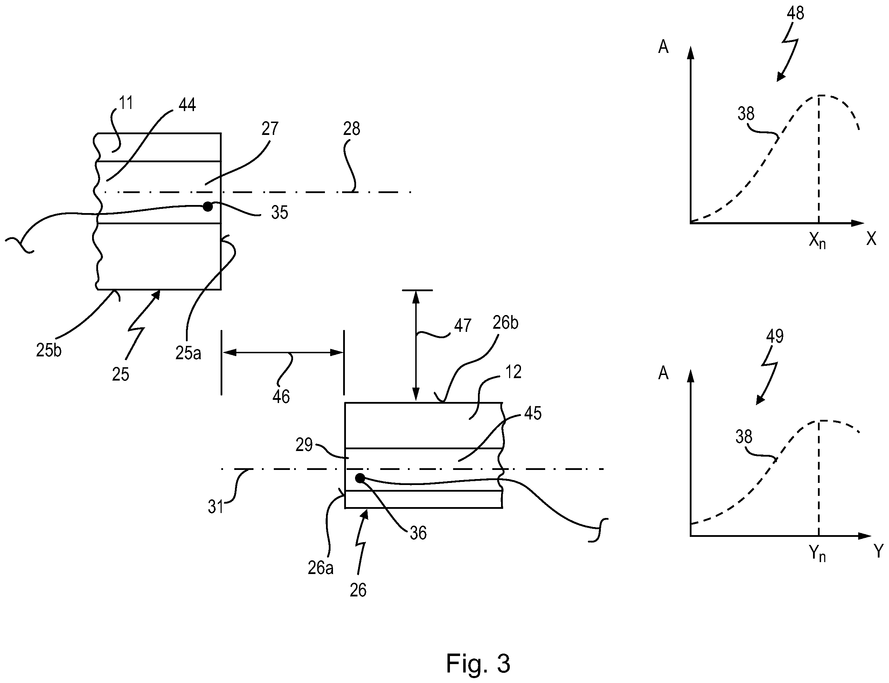

Der Unterschied in den Pulsamplituden 42 und 43 ist ein Maß für den Abstand zwischen den Öffnungen 27 und 29. Dieser Unterschied wird jetzt erfindungsgemäß verwendet, um in einem Suchverfahren sowohl den Stellweg 32 als auch den Stellweg 33 zu ermitteln, wie dies jetzt anhand der Fig. 3 erörtert wird.

In Fig. 3 sind zunächst zwei Mikrosystemkomponenten 11 und 12 jeweils im Bereich ihrer Spitzen 25 bzw. 26 gezeigt, an denen die Öffnungen 27 bzw. 29 vorgesehen sind. Diese Öffnungen 27 bzw. 29 sind Mündungen von Kanälen 44 bzw. 45, die in den Mikrosystemkomponenten 11 bzw. 12 verlaufen und an den Stirnseiten 25a bzw. 26a der Spitzen 25 bzw. 26 münden.

Der erste Kanal 44 in der Mikrosystemkomponente 11 ist beispielsweise dazu ausgelegt, auf die Öffnung 27 ein Testobjekt anzusaugen und durch Unterdruck zu halten bzw. durch Überdruck wieder abzustoßen.

Der zweite Kanal 45 ist beispielsweise dazu ausgelegt, mit Hilfe der Messelektrode 36 elektrische Messungen an der Membran eines Testobjektes oder aus dem Inneren eines Testobjektes durchzuführen. Die Spitze 26 kann auch als Kanüle ausgelegt sein, so dass über den zweiten Kanal 45 Substanzen auf ein Testobjekt oder in ein Testobjekt abgegeben bzw. aus einem Testobjekt herausgesaugt werden können, wie dies nachstehend noch weiter erörtert wird.

Die relative Positionierung der Mikrosystemkomponenten 11 und 12 zueinander entspricht der Ausgangssituation, wie sie in Fig. 1 gezeigt ist. So wie in den Fig. 1 und 3 dargestellt, werden die erste Mikrosystemkomponente 11 sowie die zweite Mikrosystemkomponente 12 in der Vorrichtung 10 montiert, wobei sich der Schlitten 16 in der Ausgangsposition befindet, in der der Referenzpunkt 23 die relative Position x1/y1 einnimmt, wie dies in dem Koordinatensystem 24 in Fig. 1 gezeigt ist.

Bei dieser Anordnung weisen die Stirnseiten 25a und 26a in x-Richtung gesehen zueinander einen Sicherheitsabstand 46 auf, während die Spitzen 25 und 26 mit ihren Längsseiten 25b und 26b zueinander in y-Richtung einen Sicherheitsabstand 47 aufweisen.

Diese Sicherheitsabstände 46 und 47 werden in Abhängigkeit von den geometrischen makroskopischen Abmaßen der verwendeten Mikrosystemkomponenten 11 und 12 so gewählt, dass keine Kollisionsgefahr besteht, wenn die Mikrosystemkomponente 12 ausgehend von der in Fig. 3 gezeigten Grundposition entweder nur in x-Richtung oder aber nur in y-Richtung verfahren wird.

Der Suchalgorithmus verfährt nun derart, dass er die Mikrosystemkomponente 12 zunächst schrittweise in x-Richtung verfährt, wobei für jede relative Position in x-Richtung (also bei gleichbleibender fester Position in y-Richtung) Messwerte 38 genommen werden.

In Fig. 3 ist ein Diagramm 48 dargestellt, das die Pulsamplituden A der Messwerte 38 für verschiedene x-Werte darstellt.

Für diesen gegebenen Sicherheitsabstand 47 in y-Richtung ergibt sich ein Maximum der Messwerte 38 bei der Koordinate xn.

Die Mikrosystemkomponente 12 wird jetzt entweder in die Ausgangsposition der Fig. 3 oder in die Nähe der grob abgeschätzten relativen Position xm für die x-Richtung gefahren und dann in entsprechender Weise schrittweise in y-Richtung bewegt, wobei auch hier bei gleichbleibend fester relativer Position in x-Richtung für jede relative Position ein Messwert 38 genommen wird.

Der Verlauf der Pulsamplituden A für verschiedene y-Werte ist in dem Diagramm 49 dargestellt. Bei yn zeigen die Messwerte 38 ein Maximum.

Aus diesen beiden Diagrammen lässt sich jetzt ablesen, dass dann, wenn die Mikrosystemkomponente 12 in die relative Position xn/yn gefahren wird, die Öffnungen 27 und 29 zumindest grob zueinander ausgerichtet sind.

Ausgehend von den groben Werten xn/yn kann das soeben beschriebene Suchverfahren wiederholt werden, wobei die Sicherheitsabstände 46 und 47 jedes Mal verringert werden, die Mikrosystemkomponente 12 jedoch nicht bis zu der relativen Position xm/ym gefahren wird.

Auf diese Weise wird iterativ die relative Position xm/ym der zweiten Mikrosystemkomponente 12 zu der ersten Mikrosystemkomponente 11 derart verfeinert, dass die Öffnungen 27 und 29 immer genauer zueinander ausgerichtet sind, wenn die Mikrosystemkomponente 12 tatsächlich in die relative Position xn/yn gefahren wird.

Diese Ausrichtung der beiden Öffnungen 27 und 29 zueinander ist in der Fig. 4 dargestellt.

In Fig. 4 ist zunächst zu erkennen, dass weder die Öffnung 27 noch die Öffnung 29 mittig in der Stirnseite 25a bzw. 25b liegen, so dass bei zueinander ausgerichteten Öffnungen 27 und 29 die Spitzen 25 und 26 versetzt zueinander angeordnet sein können.

In der in Fig. 4 gezeigten Ausrichtung sind die Öffnungen 27 und 29 im Sinne der vorliegenden Anmeldung zueinander ausgerichtet, denn die Mittelachse 28 der Öffnung 27 verläuft in der Öffnung 29, und die Mittelachse 31 der Öffnung 29 verläuft in der Öffnung 27. Im Idealfall stimmen die Mittelachsen 29 und 31 genau miteinander überein, wenn die Öffnungen 27 und 29 zueinander ausgerichtet wurden, wenn also die zweite Mikrosystemkomponente 12 in die durch das Iterationsverfahren bestimmte relative Position xn/yn zu der ersten Mikrosystemkomponente 11 verfahren wurde.

Hier sei noch erwähnt, dass der Abstand der Stirnseiten 25a und 25b zueinander jetzt im Mikrometerabstand liegt, es bleibt lediglich ein restlicher Sicherheitsabstand 50, der einen Kontakt der Stirnseiten 25a und 26a verhindert.

Der Kanal 44 der ersten Mikrosystemkomponente 11 weist übrigens einen Durchmesser 51 auf, der im Bereich von 3 bis 5 µm liegt, während der Kanal 45 in der zweiten Mikrosystemkomponente 12 einen Durchmesser 52 aufweist, der kleiner als 1 µm ist.

Beschrieben wurde das Positionierverfahren bisher nur für die x/y-Ebene, wobei selbstverständlich ein vergleichbares Iterationsverfahren auch durchgeführt wird, um die Öffnungen 27 und 29 in Richtung der z-Raumachse zueinander auszurichten.

Das Verfahren läuft entsprechend ab, die beiden Mikrosystemkomponenten 11 und 12 werden zueinander in einem Sicherheitsabstand in x- und/oder y-Richtung angeordnet, woraufhin dann die Mikrosystemkomponente 11 längs der z-Achse an der Mikrosystemkomponente 11 vorbeigefahren wird und ein Diagramm entsprechend den Diagrammen 48 und 49 für die gemessenen Pulsamplituden aufgenommen wird.

Auf diese Weise wird auch die Position zn bestimmt, in der die Öffnungen 27 und 29 in Richtung der z-Raumachse zueinander ausgerichtet sind.

Nachdem das soeben anhand der Fig. 1 bis 4 beschriebene Verfahren durchgeführt wurde, ist in dem Rechner 22 eine relative Position xn/yn/zn abgespeichert, in der die Öffnungen 27 und 29 so zueinander ausgerichtet sind, wie dies anhand der Figuren 1 bis 4 gezeigt wurde.

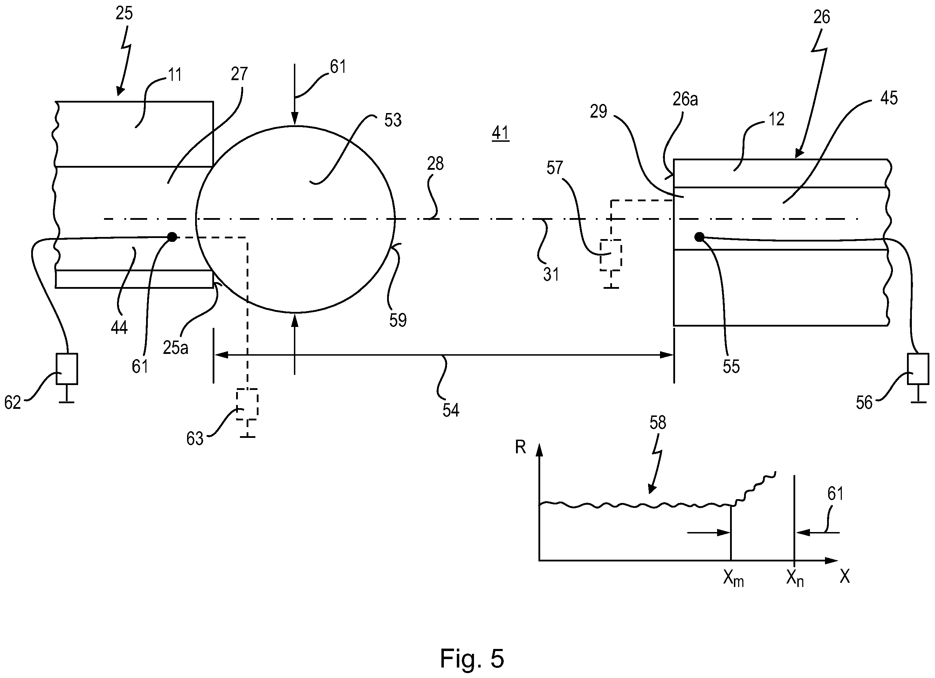

Die Vorrichtung 10 ist jetzt einsatzbereit, um ein Testobjekt 53 auf der Mikrosystemkomponente 11 zu immobilisieren und dann mit der Mikrosystemkomponente 12 zu kontaktieren, wie dies jetzt anhand von Fig. 5 erörtert werden soll.

Zu diesem Zweck werden in dem Medium 41 suspendierte Testobjekte 53 in das Reaktionsgefäß 39 eingegeben. In dem ersten Kanal 44 wird dann ein Unterdruck erzeugt, so dass an der ersten Öffnung 27 vorbei diffundierende Testobjekte 53 auf die Öffnung 27 gesaugt und dort durch Unterdruck immobilisiert, also gehalten, werden. Sollen die Testobjekte 53 wieder abgegeben werden, so wird in dem Kanal 44 ein Überdruck erzeugt.

Die Tatsache, dass an der Öffnung 27 ein Testobjekt 53 positioniert wurde, erkennt der Rechner 22 beispielsweise daran, dass der Unterdruck in dem Kanal 44 ansteigt.

Die zweite Mikrosystemkomponente 12 ist jetzt so zu der ersten Mikrosystemkomponente 11 ausgerichtet worden, dass die Mittelachsen 28 und 31 zusammenfallen bzw. so gering voneinander abweichen, dass sie durch die jeweils andere Öffnung hindurchgehen. In Richtung der x-Raumachse ist zwischen der Stirnseite 25a und 26a ein Abstand 54 vorgesehen, der mindestens das Doppelte des üblichen Durchmessers eines Objektes von der Art des Testobjektes 53 beträgt.

Das Testobjekt ist in bereits beschriebener Weise ein mikroskopisches Testobjekt, dessen üblicher Durchmesser zwischen 5 und 50 µm liegt. Das Testobjekt 53 kann beispielsweise eine biologische Zelle sein, die jetzt nach dem Patch-Clamp-Verfahren kontaktiert wird.

Um die Spitze 26 der zweiten Mikrosystemkomponente 12 jetzt auf dem Testobjekt 53 bzw. in dem Inneren des Testobjektes 53 positionieren zu können, muss zunächst der Durchmesser des Testobjektes 53 bestimmt werden.

Dazu ist in dem Kanal 45 an der Öffnung 29 in der zweiten Mikrosystemkomponente 12 eine Sensorelektrode 55 angeordnet, die mit einer Schaltung 56 verbunden ist, die ihrerseits in dem Rechner 22 integriert sein kann.

Über die Schaltung 56 kann die Sensorelektrode 55 in an sich bekannter Weise im Voltage-Clamp-Modus oder im Current-Clamp-Modus betrieben werden, so dass anhand der sich ändernden Spannung bzw. des sich ändernden Stromes der bei 57 angedeutete Widerstand vor der Mikrosystemkomponente 12 berechnet werden kann.

Wenn sich die Stirnseite 26a nun dem Testobjekt 53 nähert, so bleibt der Widerstand 57 zunächst konstant, erst unmittelbar vor Kontakt mit dem Testobjekt 53 ändert sich der Widerstandswert, er steigt beispielsweise an, wie dies in dem Diagramm 58 gezeigt ist.

Die relative x-Position der Mikrosystemkomponente 12 liegt in diesem Fall bei xm. Bei dieser relativen Position ist die Stirnseite 26a noch um die Differenz xn - xm von der Stirnseite 25a entfernt, diese Differenz entspricht also dem Durchmesser des Testobjektes 53.

Nachdem der Durchmesser des Testobjektes 53 jetzt ermittelt wurde, kann der Rechner 22 zunächst prüfen, ob überhaupt ein sinnvolles Testobjekt 53 immobilisiert wurde, oder ob es sich um Zelltrümmer oder zu große bzw. zu kleine Teilchen handelt, die aus dem Medium 41 "aufgefischt" wurden.

Ist das Testobjekt 53 nicht geeignet, wird es durch Überdruck in dem Kanal 44 wieder in das Medium 41 abgegeben, und durch Unterdruck in dem Kanal 44 wird das nächste Testobjekt 53 angesaugt.

Handelt es sich dagegen um ein taugliches Testobjekt 53, kann jetzt über die zweite Mikrosystemkomponente 12 das Testobjekt 53 an seiner Membran 59 kontaktiert werden.

In dem Rechner 22 sind sämtliche Informationen abgelegt, die erforderlich sind, damit die Spitze 26 mit ihrer Stirnseite 26a auf die Membran 59 aufgedrückt werden kann.

Die Kontrolle der Immobilisierung des Testobjektes 53 an der Öffnung 27 kann auch mit Hilfe einer Sensorelektrode 61 erfolgen, die in dem ersten Kanal 44 der ersten Mikrosystemkomponente 1 angeordnet und mit einer Schaltung 62 verbunden ist, die ihrerseits in dem Rechner 22 integriert sein kann.

Über die Schaltung 62 kann die Sensorelektrode 61 in an sich bekannter Weise im Voltage-Clamp-Modus oder im Current-Clamp-Modus betrieben werden, so dass anhand der sich ändernden Spannung bzw. des sich ändernden Stromes der bei 63 angedeutete Widerstand vor der Mikrosystemkomponente 11 berechnet werden kann.

Der zeitliche Verlauf des Widerstandswertes des Widerstandes 63 entspricht dem Verlauf gemäß Diagramm 58, nur dass auf der x-Achse jetzt nicht der gemessene Abstand sondern die Zeit aufgetragen ist.

Nachdem das Testobjekt 53 als immobilisiert erkannt wurde, kann dann in der beschriebenen Weise der Durchmesser des Testobjektes 53 bestimmt und daraus zunächst ermittelt werden, ob es sich um ein geeignetes Testobjekt 53 handelt.

Alternativ kann aber das immobilisierte Testobjekt 53 auch mit anderen Verfahren, beispielsweise auf optische oder elektrische Weise auf seine Eignung für nachfolgende Untersuchungen überprüft werden.

Wenn das Testobjekt 53 als geeignet qualifiziert wurde, wird das Verfahren fortgesetzt, ansonsten wird es verworfen und ein neues Testobjekt wird angesaugt und auf Eignung überprüft.

Je nachdem, ob dann eine Ableitung an dem von der Spitze 26 umschlossenen Membranfleck erfolgen soll, oder ob Messungen an der gesamten Zelle durchgeführt werden sollen, wird jetzt durch Anlegen eines Unterdrucks in dem Kanal 45 die Membran 59 im Bereich der Öffnung 29 perforiert, oder es wird gleich mit den Messungen begonnen, die über die Sensorelektrode 55 erfolgen.

Gleichfalls ist es jetzt möglich, die Spitze 26 teilweise in das Testobjekt 53 einzustechen, um so Substanzen in das Testobjekt 53 einzugeben bzw. aus dem Testobjekt 53 abzusaugen.

Ferner ist es möglich, die Mikrosystemkomponente 12 mit einem vorbestimmten Abstand zwischen der Membran 59 des Testobjektes 53 sowie der Stirnseite 26a der Spitze 26 anzuordnen, um dann impulsartig Substanzen an die Membran 59 abzugeben, die das Verhalten des Testobjektes 53 beeinflussen sollen.

Ferner ist es möglich, nach Herstellung eines Gigaseals in das Medium 51 Testsubstanzen einzugeben, um die Reaktion des Testobjektes 53 auf die Testsubstanzen zu messen.

Für diese Messungen wird die Sensorelektrode 55 verwendet, die identisch zu der Messelektrode 36 ist. Gleichfalls ist die Sensorelektrode 61 identisch zu der Signalelektrode 35.

Nach/vor oder während dieser Messung kann das Testobjekt 53 an die zweite Öffnung 29 übergeben und von der ersten Öffnung 27 wegbewegt werden. Das Testobjekt 53 kann wegen der bekannten relativen geometrischen Bedingungen in einem definierten Abstand zu der ersten Öffnung 27 positioniert werden, aus der dann Testsubstanzen auf das Testobjekt 53 abgegeben werden. Mit der Elektrode 36/55 können dann die Reaktionen des Testobjektes 53 auf diese Testsubstanzen gemessen werden.

Selbstverständlich ist es auch möglich, nicht nur eine zweite Mikrosystemkomponente 12 sondern mehrere Mikrosystemkomponenten 12 einzusetzen, für deren Öffnungen jeweils die relative Position zu der Öffnung in der ersten Mikrosystemkomponente 11 bestimmt wird.

Dabei ist es auch möglich, die zum Halten des Testobjektes 53 vorgesehen Mikrosystemkomponente auf dem Schlitten 16 anzuordnen und in dem Reaktionsgefäß 39 mehrere Mikrosystemkomponenten anzuordnen, die der Kontaktierung im Patch-Clamp-Modus sowie dem Umspülen mit Testsubstanzen dienen.

In einem solchen Fall muss zunächst für die auf dem Schlitten 16 angeordnete Mikrosystemkomponente in jedem Einzelfall die relative Position bestimmt werden, in der die Öffnung dieser Mikrosystemkomponente zu jeder der Öffnungen der anderen Mikrosystemkomponenten ausgerichtet ist. Der Durchmesser eines aufgefangenen Testobjektes muss jedoch nur einmal bestimmt werden, er kann dann bei dem Anfahren an die anderen Mikrosystemkomponenten jeweils berücksichtigt werden, da im Gegensatz zu den relative Positionen der unterschiedlichen Öffnungen zueinander der Durchmesser absolut bestimmt wird.

Auf diese Weise schafft die erfindungsgemäße Vorrichtung die Möglichkeit, das erfindungsgemäße Verfahren durchzuführen und somit in vollständig automatisierter Form an verschiedenen Testobjekten nacheinander Messungen und/oder Manipulationen durchzuführen.

Die genaue Bestimmung der relativen Positionen der einzelnen Mikrosystemkomponenten zueinander, in die sie gebracht werden müssen, damit die entsprechenden Manipulationen und/oder Messungen durchgeführt werden können, erfolgt jeweils dann neu und auf automatisierte Weise, wenn neue Mikrosystemkomponenten 11, 12 in die Vorrichtung 10 montiert wurden.

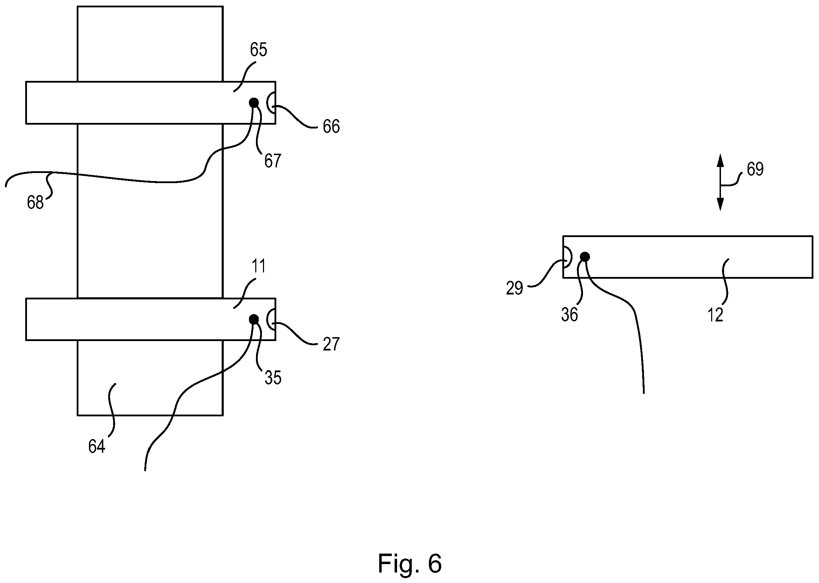

Des weiteren kann auf einem Träger 64 eine dritte Mikrosystemkomponente 65 mit einer dritten Öffnung 66 mechanisch fest mit der ersten Mikrosystemkomponente 11 verbunden sein, wie es schematisch in Fig. 6 gezeigt ist.

In der Öffnung 66 ist eine Elektrode 67 angeordnet, die über ein Kabel 68 mit dem Rechner 22 aus Fig. 1 verbunden ist. Die Elektrode 67 kann wie die Signalelektrode 35 und/oder wie die Sensorelektrode 61 verwendet werden, um die relative Position zwischen der Öffnung 66 und der Öffnung 29 in der beschriebenen Weise zu bestimmen und/oder um zu erkennen, ob ein Testobjekt an der Öffnung 66 immobilisiert wurde.

Die zweite Mikrosystemkomponente 12 ist relativ zu den beiden anderen Mikrosystemkomponenten 11, 65 verfahrbar, was durch einen Pfeil 69 angedeutet ist. Auf diese Weise kann ein an der ersten Öffnung 27 immobilisiertes Testobjekt von der zweiten Mikrosystemkomponente 12 übernommen und zu der dritten Mikrosystemkomponente 65 transportiert werden, wo dann Testsubstanzen aus der dritten Öffnung 66 auf das Testobjekt gegeben werden können. Mit der Elektrode 36/55 werden dann die Reaktionen des Testobjektes auf die Testsubstanzen erfasst.

In all diesen Anwendungen dient die erste Mikrosystemkomponente 11 dazu, ein Testobjekt aus einer Suspension heraus immobilisieren und darauf überprüfen zu können, ob es für die geplanten Untersuchungen geeignet ist. Wird das Testobjekt als geeignet qualifiziert, so kann es mit der Mikrosystemkomponente 12 kontaktiert und ggf. von der ersten Mikrosystemkomponente 11 wegbewegt werden. Testsubstanzen können dann aus der ersten uns/oder einer dritten Mikrosystemkomponente 11, 65 auf das Testobjekt gegeben werden.

Damit ist es möglich, aus einer Suspension mit nur wenigen geeigneten Testobjekten in reproduzierbarer und verlässlicher Weise schnell ein geeignetes Testobjekt herauszusuchen und dieses dann den Messungen zuzuführen.

Diese Verfahren sind nur möglich, weil gemäß dem neuen Verfahren die relativen Positionen der Öffnungen 27, 29 und 66 zueinander automatisch und mit hoher Genauigkeit bestimmt werden können.

## Patentansprüche

1. Verfahren zur automatisierten Bestimmung der relativen Position (x/y/z) zwischen einer ersten Öffnung (27) an einer ersten Mikrosystemkomponente (11), die mit einem in der ersten Öffnung (27) mündenden ersten Kanal (44) versehenen ist, und zumindest einer zweiten Öffnung (29) an einer zweiten Mikrosystemkomponente (12), die mit einem in der zweiten Öffnung (29) mündenden zweiten Kanal (45) versehenen ist, bei dem sich die beiden Mikrosystemkomponenten (11, 12) zumindest im Bereich (25, 26) der Öffnungen (27, 29) in einem flüssigen Medium 841) befinden, mit den Schritten
- unter der Kontrolle einer über einen Rechner (22) gesteuerten Steuervorrichtung (15) werden die erste und die zweite Mikrosystemkomponente (11, 12) relativ zueinander im Raum in unterschiedliche relative Positionen (x/y/z) zueinander verfahren,
- an einer der beiden Mikrosystemkomponenten (12, 12) werden elektrische Signale (37) an der Öffnung (27) durch eine Signalelektrode (35) abgegeben, die an der anderen der beiden Mikrosystemkomponenten (11, 12) als Messwerte (38) an der Öffnung (29) durch eine Messelektrode (36) aufgenommen werden, die von der relativen Position der beiden Mikrosystemkomponenten (11, 12) zueinander abhängen,
- für unterschiedliche relative Positionen (x/y/z) zwischen den beiden Mikrosystemkomponenten (11, 12) werden Messwerte (38) bestimmt, aus denen in der Steuervorrichtung (15) ermittelt wird, in welcher relativen Position (xn/yn/zn) die beiden Mikrosystemkomponenten (11, 12) so zueinander zu positionieren sind, dass ihre Öffnungen (27, 29) zueinander ausgerichtet sind, sodass die beiden Öffnungen (27,29) einander derart gegenüberliegen, dass ihre Mittenachsen in der jeweils anderen Öffnung verlaufen.

2. Verfahren nach Anspruch 1, **dadurch gekennzeichnet, dass** die elektrischen Signale (37) über die in dem ersten Kanal (44) angeordnete Signalelektrode (35) in das Medium (41) abgegeben und die Messwerte (38) über die in dem zweiten Kanal (45) angeordnete Messelektrode (36) aufgenommen werden.

3. Verfahren nach Anspruch 2, **dadurch gekennzeichnet, dass** die elektrischen Signale (37) in das Medium (41) abgegebene Spannungsimpulse sind, deren Pulsamplituden (42, 43) an der zweiten Mikrosystemkomponente (12) als Messwerte (38) erfasst werden

4. Verfahren nach einem der Ansprüche 1 bis 3, **dadurch gekennzeichnet, dass** die beiden Mikrosystemkomponenten (11, 12) in zumindest zwei zueinander orthogonalen Raumachsen (x, y) zueinander verfahren werden, wobei die erste Raumachse (x) etwa parallel zu den Projektionen der Längsachsen (28, 31) der beiden Öffnungen (27, 29) in die durch die beiden Raumachsen (x, y) aufgespannte Ebene verläuft.

5. Verfahren nach Anspruch 4, **dadurch gekennzeichnet, dass** in der ersten Raumachse (x) eine grobe relative Position (xn) bestimmt wird, indem bei fester relativer Position (y1) in der zweiten Raumachse (y) die beiden Mikrosystemkomponenten (11, 12) in der ersten Raumachse (x) in verschiedene relative Positionen gefahren und für jede relative Position ein Messwert (38) genommen wird.

6. Verfahren nach Anspruch 5, **dadurch gekennzeichnet, dass** die feste relative Position (y1) in der zweiten Raumachse (y) so gewählt wird, dass zwischen den beiden Mikrosystemkomponenten (11, 12) in Richtung der zweiten Raumachse (y) ein Sicherheitsabstand (47) besteht, der in Abhängigkeit von den Abmaßen der Mikrosystemkomponenten (11, 12) in Richtung der zweiten Raumachse (y) bestimmt wird.

7. Verfahren nach Anspruch 4, **dadurch gekennzeichnet, dass** in der zweiten Raumachse (y) eine grobe relative Position (yn) bestimmt wird, indem bei fester relativer Position (x1; xn) in der ersten Raumachse (x) die beiden Mikrosystemkomponenten (11, 12) in der zweiten Raumachse (y) in verschiedene relative Positionen gefahren und für jede relative Position ein Messwert (38) genommen wird.

8. Verfahren nach Anspruch 6 und 7, **dadurch gekennzeichnet, dass** die feste relative Position (x1) in der ersten Raumachse (x) so gewählt wird, dass zwischen den beiden Öffnungen (27, 29) in Richtung der ersten Raumachse (x) ein Sicherheitsabstand (46) besteht, der in Abhängigkeit von der groben relativen Position (xn) in der ersten Raumrichtung (x) bestimmt wird.

9. Verfahren nach Anspruch 7 oder 8, **dadurch gekennzeichnet, dass** in der ersten Raumachse (x) eine feinaufgelöste relative Position (xn) bestimmt wird, indem bei grober relativer Position (yn) in der zweiten Raumachse (y) die beiden Mikrosystemkomponenten (11, 12) in der ersten Raumachse (x) in verschiedene relative Positionen gefahren werden und für jede relative Position ein Messwert (38) genommen wird.

10. Verfahren nach Anspruch 9, **dadurch gekennzeichnet, dass** in der zweiten Raumachse (y) eine feinaufgelöste relative Position (yn) bestimmt wird, indem bei feinaufgelöster relativer Position (xn) in der ersten Raumachse (x) die beiden Mikrosystemkomponenten (11, 12) in der zweiten Raumachse (y) in verschiedene relative Positionen gefahren werden und für jede relative Position ein Messwert (38) genommen wird.

11. Verfahren nach einem der Ansprüche 4 bis 10, **dadurch gekennzeichnet, dass** die beiden Mikrosystemkomponenten (11, 12) in einer dritten Raumachse (z) relativ zueinander verfahren werden, die orthogonal zu der ersten und zweiten Raumachse (x, y) verläuft, wobei in der dritten Raumachse (z) eine relative Position bestimmt wird, indem bei fester relativer Position in der ersten und zweiten Raumachse (x, y) die beiden Mikrosystemkomponenten (11, 12) in der dritten Raumachse 8z) in verschiedene relative Positionen gefahren werden und für jede relative Position ein Messwert (38) genommen wird.

12. Verfahren nach einem der Ansprüche 1 bis 11, **dadurch gekennzeichnet, dass** die erste Mikrosystemkomponente (11) zum Immobilisieren einer biologischen Zelle und die zweite Mikrosystemkomponente (12) zum Kontaktieren der so immobilisierten Zelle ausgerüstet ist.

13. Verfahren nach einem der Ansprüche 1 bis 12, **dadurch gekennzeichnet, dass** eine dritte Mikrosystemkomponente mit einer dritten Öffnung vorgesehen ist, wobei die dritte Mikrosystemkomponente vorzugsweise mit der ersten Mikrosystemkomponente mechanisch fest verbunden ist, und dass die relative Position zwischen der zweiten und der dritten Öffnung bestimmt wird.

14. Verfahren zum automatisierten Kontaktieren von zumindest einem mikroskopischen Testobjekt (53), das an einer Öffnung (27) an einer ersten Mikrosystemkomponente (11) immobilisiert und dann über eine Öffnung (29) an einer zweiten Mikrosystemkomponente (12) kontaktiert wird, bei dem
- mit dem Verfahren nach einem der Ansprüche 1 bis 13 die relative Position zwischen den beiden Öffnungen bestimmt wird,
- an der ersten Öffnung (27) ein mikroskopisches Testobjekt (53) immobilisiert wird,
- wobei vorzugsweise das Testobjekt auf seine Eignung für nachfolgende Untersuchungen überprüft wird,
- die beiden Öffnungen (27, 29) längs einer ersten Raumachse (x) zueinander mit einem Abstand (54) ausgerichtet werden, der mindestens dem doppelten maximalen Durchmesser eines zu kontaktierenden mikroskopischen Testobjekts (53) entspricht,
- mittels einer Sensorelektrode (55) der Widerstand (57) vor der zweiten Öffnung (29) gemessen wird,
- in einem Suchverfahren der Abstand (54) zwischen den beiden Öffnungen (27, 29) in der ersten Raumachse (x) schrittweise reduziert und für jeden neuen Abstand erneut der Widerstand (57) gemessen wird,
- wobei bei einer vorgegebenen Änderung des gemessenen Widerstandswertes das Suchverfahren beendet und das mikroskopische Testobjekt (53) als kontaktiert bewertet wird.

15. Verfahren nach Anspruch 14, **dadurch gekennzeichnet, dass** während des Immobilisierens des Testobjektes (53) an der ersten Öffnung (27) mittels einer Sensorelektrode der Widerstand vor der ersten Öffnung (27) gemessen wird.

16. Verwendung einer Vorrichtung zum automatisierten Kontaktieren von zumindest einem mikroskopischen Testobjekt (53), das an einer ersten Öffnung (27) immobilisiert und dann über eine zweite Öffnung (29) kontaktiert wird, für das Verfahren nach einem der Ansprüche 1 bis 15, wobei die Vorrichtung
- zumindest eine erste Mikrosystemkomponente (11), an der die erste Öffnung (27) vorgesehen ist, die mit einem in der ersten Öffnung (27) mündenden ersten Kanal (44) versehen ist,
- zumindest eine zweite Mikrosystemkomponente (12), an der die zweite Öffnung (29) vorgesehen ist, die mit einem in der zweiten Öffnung (29) mündenden zweiten Kanal(45) versehen ist,
- ein Reaktionsgefäß (39) zur Aufnahme eines flüssigen Mediums (41), wobei die beiden Mikrosystemkomponenten (11, 12) zumindest im Bereich (25, 26) der Öffnungen (27, 29) in das Medium (41) eingetaucht sind,
- eine Mechanik (14), über die die beiden Mikrosystemkomponenten (11, 12) relativ zueinander zumindest in zwei zueinander orthogonalen Raumrichtungen (x, y) verfahrbar sind,
- eine Steuervorrichtung (15, 22), über die die Mechanik (14) automatisiert gesteuert wird, und
- eine Messanordnung (34) aufweist, über die durch eine Signalelektrode (35) an der Öffnung (27) an einer der beiden Mikrosystemkomponenten (11,12) elektrische Signale (37) abgegeben werden, die durch eine Messelektrode (36) an der Öffnung (29) an einer der beiden Mikrosystemkomponenten (11,12) als Messwerte (38) aufgenommen werden, die von der relativen Position der beiden Mikrosystemkomponenten (11, 12) zueinander abhängen, wobei
- die Steuervorrichtung (15, 22) dazu eingerichtet ist, aus Messwerten (38) für unterschiedliche relative Positionen (x/y/z) zwischen den beiden Mikrosystemkomponenten (11, 12) zu ermitteln, in welcher relativen Position (xn/yn/zn) die beiden Mikrosystemkomponenten (11, 12) so zueinander zu positionieren sind, dass ihre Öffnungen (27, 29) zueinander ausgerichtet sind, sodass die beiden Öffnungen (27,29) einander derart gegenüberliegen, dass ihre Mittenachsen in der jeweils anderen Öffnung verlaufen.

## Claims

1. A method for automated determination of the relative position (x/y/z) between a first hole (27) on a first microsystem component (11), which is provided with a first channel (44) opening in the first hole (27), and at least one second hole (29) on a second microsystem component (12), which is provided with a second channel (45) opening in the second hole (29), wherein the two microsystem components (11, 12) are located in a liquid medium (41) at least in the region (25, 26) of the holes (27, 29), comprising the steps:
- under the supervision of a control device (15) controlled by a computer (22), the first and second microsystem components (11, 12) are displaced relative to one another into different relative positions (x/y/z),
- on one of the two microsystem components (12, 12) electrical signals are delivered at the hole (27) by a signal electrode (35) and are recorded on the other of the two microsystem components (11, 12) as measurement values (38) at the hole (29) by a measurement electrode (36), which measurement values depend on the relative position of the two microsystem components (11, 12) with respect to one another,
- for different relative positions (x/y/z) between the two microsystem components (11, 12), measurement values (38) are determined, from which in the control device (15) the relative position (xn/yn/zn) is ascertained into which the two microsystem components (11, 12) are to be positioned with respect to one another in such a way that their holes (27, 29) are mutually aligned, such that the two holes (27, 29) lie opposite one another such that their middle axis extend in the other respective hole.

2. The method as claimed in claim 1, **characterized in that** the electrical signals (37) are delivered into the medium (41) by means of the signal electrode (35) arranged in the first channel (44), and the measurement values (38) are recorded by means of the measurement electrode (36) arranged in the second channel (45).

3. The method as claimed in claim 2, **characterized in that** the electrical signals (37) are voltage pulses delivered into the medium (41), the pulse amplitudes (42, 43) of which are acquired as measurement values (38) at the second microsystem component (12).

4. The method as claimed in any one of claims 1 to 3, **characterized in that** the two microsystem components (11, 12) are displaced with respect to one another along at least two mutually orthogonal spatial axes (x, y), the first spatial axis (x) extending approximately parallel to the projections of the longitudinal axes (28, 31) of the two holes (27, 29) into the plane spanned by the two spatial axes (x, y).

5. The method as claimed in claim 4, **characterized in that** a rough relative position (xn) is determined along the first spatial axis (x) by displacing the two microsystem components (11, 12) along the first spatial axis (x) into different relative positions while maintaining a fixed relative position (y1) along the second spatial axis (y) and taking a measurement value (38) for each relative position.

6. The method as claimed in claim 5, **characterized in that** the fixed relative position (y1) along the second spatial axis (y) is selected in such a way that there is a safety distance (47), which is determined as a function of the dimensions of the microsystem components (11, 12) in the direction of the second spatial axis (y), between the two microsystem components (11, 12) in the direction of the second spatial axis (y).

7. The method as claimed in claim 4, **characterized in that** a rough relative position (yn) is determined along the second spatial axis (y) by displacing the two microsystem components (11, 12) along the second spatial axis (y) into different relative positions while maintaining a fixed relative position (x1; xn) along the first spatial axis (x) and taking a measurement value (38) for each relative position.

8. The method as claimed in claims 6 and 7, **characterized in that** the fixed relative position (x1) along the first spatial axis (x) is selected in such a way that there is a safety distance (46), which is determined as a function of the rough relative position (xn) along the first spatial direction (x), between the two holes (27, 29) in the direction of the first spatial axis (x).

9. The method as claimed in claim 7 or 8, **characterized in that** a finely resolved relative position (xn) is determined along the first spatial axis (x) by displacing the two microsystem components (11, 12) along the first spatial axis (x) into different relative positions while maintaining a rough relative position (yn) along the second spatial axis (y) and taking a measurement value (38) for each relative position.

10. The method as claimed in claim 9, **characterized in that** a finely resolved relative position (yn) is determined along the second spatial axis (y) by displacing the two microsystem components (11, 12) along the second spatial axis (y) into different relative positions while maintaining a finely resolved relative position (xn) along the first spatial axis (x) and taking a measurement value (38) for each relative position.

11. The method as claimed in any one of claims 4 to 10, **characterized in that** the two microsystem components (11, 12) are displaced relative to one another along a third spatial axis (z) which extends orthogonally to the first and second spatial axes (x, y), a relative position being determined in the third spatial axis (z) by displacing the two microsystem components (11, 12) along the third spatial axis (z) into different relative positions while maintaining a fixed relative position along the first and second spatial axes (x, y) and taking a measurement value (38) for each relative position.

12. The method as claimed in any one of claims 1 to 11, **characterized in that** the first microsystem component (11) is configured for immobilizing a biological cell and the second microsystem component (12) is configured for contacting the cell immobilized in this way.

13. The method as claimed in any one of claims 1 to 12, **characterized in that** a third microsystem component having a third hole is provided, the third microsystem component preferably being mechanically connected in a fixed way to the first microsystem component, and **in that** the relative position between the second and third holes is determined.

14. A method for the automated contacting of at least one microscopic test object (53) which is immobilized at a hole (27) on a first microsystem component (11) and is then contacted through a hole (29) on a second microsystem component (12), wherein
- the relative position between the two holes is determined with the method as claimed in any one of claims 1 to 13,
- a microscopic test object (53) is immobilized on the first hole (27),
- the test object preferably being checked for its suitability for subsequent examinations,
- the two holes (27, 29) are mutually aligned along a first spatial axis (x) with a distance (54) which corresponds to at least two times the maximum diameter of a microscopic test object (53) to be contacted,
- the resistance (57) in front of the second hole (29) is measured by means of a sensor electrode (55),
- in a search method, the distance (54) between the two holes (27, 29) along the first spatial axis (x) is reduced stepwise and the resistance (57) is measured again for each new distance,
- the search method being ended, and the microscopic test object (53) being evaluated as contacted, when there is a predetermined change in the measured resistance value.

15. The method as claimed in claim 14, **characterized in that** the resistance in front of the first hole (27) is measured by means of a sensor electrode during the immobilization of the test object (53) on the first hole (27).

16. Use of a device for the automated contacting of at least one microscopic test object (53) which is immobilized on a first hole (27) and is then contacted through a second hole (29), for the method of any one claims 1 through 15, wherein the device comprises
- at least one first microsystem component (11), on which the first hole (27) is provided and which is provided with a first channel (44) opening in the first hole (27),
- at least one second microsystem component (12), on which the second hole (29) is provided and which is provided with a second channel (45) opening in the second hole (29),
- a reaction vessel (39) for holding a liquid medium (41), the two microsystem components (11, 12) being immersed in the medium (41) at least in the region (25, 26) of the holes (27, 29),
- a mechanism (14), by means of which the two microsystem components (11, 12) can be displaced relative to one another at least in two mutually orthogonal spatial directions (x, y),
- a control device (15, 22), by means of which the mechanism (14) is controlled in an automated fashion, and
- a measurement arrangement (34), by means of which electrical signals (37) are delivered by a signal electrode (35) at the hole (27) of one of the two microsystem components (11, 12) and are recorded by a measurement electrode (36) at the hole (29) on the other of the two microsystem components (11, 12) as measurement values (38) which depend on the relative position of the two microsystem components (11, 12) with respect to one another,
- the control device (15, 22) being adapted in order to ascertain, from measurement values (38) for different relative positions (x/y/z) between the two microsystem components (11, 12), the relative position (xn/yn/zn) in which the two microsystem components (11, 12) are to be positioned with respect to one another in such a way that their holes (27, 29) are mutually aligned, such that the two holes (27, 29) lie opposite one another such that their middle axis extend in the other respective hole.

## Revendications

1. Procédé pour la détermination automatisée de la position relative (x/y/z) entre une première ouverture (27) aménagée sur un premier composant de microsystème (11) avec un premier canal (44) débouchant dans la première ouverture (27), et au moins une deuxième ouverture (29) aménagée sur un deuxième composant de microsystème (12) qui est avec un deuxième canal (45) débouchant dans la deuxième ouverture (29), selon lequel les deux composants de microsystème (11, 12) se trouvent au moins dans la zone (25, 26) des ouvertures (27, 29) dans un milieu liquide (41), avec les étapes
- le premier et le deuxième composant de microsystème (11, 12) sont déplacés l'un par rapport à l'autre dans l'espace en différentes positions relatives (x/y/z) les unes par rapport aux autres sous la vérification d'un dispositif de commande (15) commandé par un ordinateur (22),
- sur l'un des deux composants du microsystème (12, 12), des signaux électriques (37) sont émis au niveau de l'ouverture (27) par une électrode de signal (35), qui sont reçus sur l'autre des deux composants de microsystème (11, 12) au niveau de l'ouverture (29) par une électrode de mesure (36) en tant que valeurs de mesure (38), qui dépendent de la position relative des deux composants de microsystème (11, 12) l'un par rapport à l'autre,
- des valeurs de mesure (38) sont déterminées pour différentes positions relatives (x/y/z) entre les deux composants de microsystème (11, 12) desquelles est déduit dans le dispositif de commande (15) en quelle position relative (xn/yn/zn) les deux composants de microsystème (11, 12) doivent être positionnés l'un par rapport à l'autre afin que leurs ouvertures (27, 29) soient orientées de telle sorte que les deux ouvertures (27, 29) se font face pour que leurs axes médians passent dans l'autre ouverture respective.

2. Procédé selon la revendication 1, **caractérisé en ce que** les signaux électriques (37) sont émis dans le milieu (41) par le biais des électrodes de signal (35) disposées dans le premier canal (44) et les valeurs de mesure (38) sont reçues par le biais de l'électrode de mesure (36) disposée dans le deuxième canal (45).

3. Procédé selon la revendication 2, **caractérisé en ce que** les signaux électriques (37) sont des impulsions de tension émises dans le milieu (41), dont les amplitudes (42, 43) sont captées sur le deuxième composant de microsystème (12) en tant que valeurs de mesure (38).

4. Procédé selon l'une des revendications 1 à 3, **caractérisé en ce que** les deux composants de microsystème (11, 12) sont déplacés dans au moins deux axes spatiaux (x, y) orthogonaux l'un par rapport à l'autre, dans lequel le premier axe spatial (x) est à peu près parallèle aux projections des axes longitudinaux (28, 31) des deux ouvertures (27, 29) dans le plan s'étendant à travers les deux axes spatiaux (x, y).

5. Procédé selon la revendication 4, **caractérisé en ce que**, dans le premier axe spatial (x), une position relative grossière (xn) est déterminée, dans laquelle sont déplacés les deux composants de microsystème (11, 12) dans le premier axe spatial (x) dans différentes positions relatives lorsque la position relative (y1) dans le deuxième axe spatial (y) est fixe et une valeur de mesure (38) est prise pour chaque position relative.

6. Procédé selon la revendication 5, **caractérisé en ce que** la position relative (y1) fixe est choisie dans le deuxième axe spatial (y) de telle sorte que, entre les deux composants de microsystème (11, 12) dans le sens du deuxième axe spatial (y), il existe une distance de sécurité (47) qui est déterminée en fonction des dimensions des composants de microsystème (11, 12) dans le sens du deuxième axe spatial (y).

7. Procédé selon la revendication 4, **caractérisé en ce que**, dans le deuxième axe spatial (y), une position relative grossière (yn) est déterminée, dans laquelle sont déplacés les deux composants de microsystème (11, 12) dans le deuxième axe spatial (y) dans différentes positions relatives lorsque la position relative (x1 ; xn) dans le premier axe spatial (x) est fixe et une valeur de mesure (38) est prise pour chaque position relative.

8. Procédé selon la revendication 6 et 7, **caractérisé en ce que** la position relative fixe (x1) dans le premier axe spatial (x) est choisie de telle sorte que, entre les deux ouvertures (27, 29) dans le sens du premier axe spatial (x), il existe une distance de sécurité (46) qui est déterminée en fonction de la position relative grossière (xn) dans le sens du premier axe spatial (x).

9. Procédé selon la revendication 7 ou 8, **caractérisé en ce que**, dans le premier axe spatial (x), une position relative à fine résolution (xn) est déterminée, dans laquelle sont déplacés les deux composants de microsystème (11, 12) dans le premier axe spatial (x) dans différentes positions relatives lorsque la position relative (yn) dans le deuxième axe spatial (y) est grossière et une valeur de mesure (38) est prise pour chaque position relative.

10. Procédé selon la revendication 9, **caractérisé en ce que**, dans le deuxième axe spatial (y), une position relative à fine résolution (yn) est déterminée dans laquelle sont déplacés les deux composants de microsystème (11, 12) dans le deuxième axe spatial (y) dans différentes positions relatives lorsque la position relative (xn) dans le premier axe spatial (x) est à fine résolution et une valeur de mesure (38) est prise pour chaque position relative.

11. Procédé selon l'une des revendications 4 à 10, **caractérisé en ce que** les deux composants de microsystème (11, 12) sont déplacés l'un par rapport à l'autre dans le troisième axe spatial (z) qui s'étend de manière orthogonale par rapport au premier et au deuxième axe spatial (x, y), dans lequel une position relative est déterminée dans le troisième axe spatial (z), dans laquelle sont déplacés les deux composants de microsystème (11, 12) dans le troisième axe spatial (z) dans différentes positions relatives lorsque la position relative dans le premier et le deuxième axe spatial (x, y) est fixe et une valeur de mesure (38) est prise pour chaque position relative.

12. Procédé selon l'une des revendications 1 à 11, **caractérisé en ce que** le premier composant de microsystème (11) est équipé pour l'immobilisation d'une cellule biologique et le deuxième composant de microsystème (12) pour la mise en contact de la cellule ainsi immobilisée.

13. Procédé selon l'une des revendications 1 à 12, **caractérisé en ce qu'**un troisième composant de microsystème est doté d'une troisième ouverture, dans lequel le troisième composant de microsystème est lié de manière fixe mécaniquement de préférence au premier composant de microsystème et que la position relative entre la deuxième et la troisième ouverture est déterminée.

14. Procédé pour la mise en contact automatisée d'au moins un objet test microscopique (53) qui est immobilisé au niveau d'une ouverture (27) sur un premier composant de microsystème (11) et est ensuite mis en contact par le biais d'une ouverture (29) sur un deuxième composant de microsystème (12), selon lequel
- la position relative entre les deux ouvertures est déterminée avec le procédé selon l'une des revendications 1 à 13,
- un objet de test microscopique (53) est immobilisé au niveau de la première ouverture (27),
- dans lequel l'objet de test est de préférence vérifié sur son adéquation pour les analyses suivantes,
- les deux ouvertures (27, 29) sont orientées le long d'un premier axe spatial (x) l'une par rapport à l'autre à une distance (54) qui correspond au moins au double du diamètre maximal d'un objet de test microscopique (53) à mettre en contact,
- la résistance (57) est mesurée au moyen d'une électrode de détection (55) devant la deuxième ouverture (29),
- dans un procédé à tâtons, la distance (54) entre les deux ouvertures (27, 29) est réduite pas à pas dans le premier axe spatial (x) et la résistance (57) est de nouveau mesurée pour chaque nouvelle distance,
- dans lequel lors d'une modification prédéfinie de la valeur de résistance mesurée, le procédé à tâtons se termine et l'objet de test microscopique (53) est évalué comme étant mis en contact.

15. Procédé selon la revendication 14, **caractérisé en ce que**, pendant l'immobilisation de l'objet de test (53) au niveau de la première ouverture (27), la résistance est mesurée au moyen d'une électrode de détection avant la première ouverture (27).

16. Utilisation d'un dispositif pour la mise en contact automatisée d'au moins un objet de test microscopique (53) qui est immobilisé au niveau d'une première ouverture (27) et est ensuite mis en contact par le biais d'une deuxième ouverture (29), pour le procédé selon l'une des revendications 1 à 15, dans laquelle le dispositif présente
- au moins un premier composant de microsystème (11) sur lequel est aménagée la première ouverture (27) qui est dotée d'un premier canal (44) débouchant dans la première ouverture (27),
- au moins un deuxième composant de microsystème (12) sur lequel est aménagée la deuxième ouverture (29) qui est dotée d'un deuxième canal (45) débouchant dans la deuxième ouverture (29),
- une cuve de réaction (39) pour la réception d'un milieu liquide (41), dans lequel les deux composants de microsystème (11, 12) sont plongés au moins dans la zone (25, 26) des ouvertures (27, 29) dans le milieu (41),
- une mécanique (14) par le biais de laquelle les deux composants de microsystème (11, 12) peuvent être déplacés l'un par rapport à l'autre au moins dans deux sens spatiaux (x, y) orthogonaux l'un par rapport à l'autre,
- un dispositif de commande (15, 22) par le biais duquel la mécanique (14) est commandée de manière automatisée, et
- une installation de mesure (34) par le biais de laquelle des signaux électriques (37) sont émis par une électrode de signal (35) au niveau de l'ouverture (27) sur l'un des deux composants de microsystème (11, 12), signaux électriques qui sont reçus sur l'un des deux composants de microsystème (11, 12) au niveau de l'ouverture (29) par une électrode de mesure (36) en tant que valeurs de mesure (38) et dépendent de la position relative des deux composants de microsystème (11, 12) l'un par rapport à l'autre, dans lequel
- le dispositif de commande (15, 22) est conçu pour déterminer, à partir des valeurs de mesure (38) pour les différentes positions relatives (x/y/z) entre les deux composants de microsystème (11, 12), en quelle position relative (xn/yn/zn) les deux composants de microsystème (11, 12) doivent être positionnés l'un par rapport à l'autre afin que leurs ouvertures (27, 29) soient orientées de telle sorte que les deux ouvertures (27, 29) se font face pour que leurs axes médians passent dans l'autre ouverture respective.
